(19) Europäisches Patentamt / European Patent Office / Office européen des brevets

(11) EP 4 434 520 A1

(12) EUROPEAN PATENT APPLICATION
published in accordance with Art. 153(4) EPC

(43) Date of publication:
25.09.2024 Bulletin 2024/39

(21) Application number: 22895692.6

(22) Date of filing: 18.11.2022

(51) International Patent Classification (IPC):
A61K 31/4184 (2006.01) A61P 3/06 (2006.01)
A61P 3/10 (2006.01) A61P 7/00 (2006.01)
A61P 9/00 (2006.01) A61P 9/04 (2006.01)
A61P 9/06 (2006.01) A61P 9/10 (2006.01)
A61P 13/12 (2006.01) A61P 21/00 (2006.01)
A61P 43/00 (2006.01)

(52) Cooperative Patent Classification (CPC):
A61K 31/4184; A61P 3/06; A61P 3/10; A61P 7/00; A61P 9/00; A61P 9/04; A61P 9/06; A61P 9/10; A61P 13/12; A61P 21/00; A61P 43/00

(86) International application number:
PCT/JP2022/042796

(87) International publication number:
WO 2023/090411 (25.05.2023 Gazette 2023/21)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 19.11.2021 JP 2021188247

(71) Applicant: Shionogi & Co., Ltd.
Osaka-shi, Osaka 541-0045 (JP)

(72) Inventors:
- YOSHIDA, Yasunobu
Osaka 541-0045 (JP)
- UNO USUI, Mai
Osaka 541-0045 (JP)
- SHIMAZAKI, Atsuyuki
Osaka 541-0045 (JP)
- YUKIOKA, Hideo
Osaka 541-0045 (JP)
- KASHIWAGI, Yuto
Osaka 541-0045 (JP)
- YAMADA, Tomomi
Osaka 541-0045 (JP)

(74) Representative: Vossius & Partner
Patentanwälte Rechtsanwälte mbB
Siebertstrasse 3
81675 München (DE)

# (54) PHARMACEUTICAL FOR TREATING HEART DISEASE OR SKELETAL MUSCLE DISEASE

(57) Provided is a pharmaceutical composition for treating and/or preventing at least one of heart diseases, heart disease complications, skeletal muscle diseases, and skeletal muscle conditions, the pharmaceutical composition having an excellent ACC2-selective inhibitory effect and having no side effects such as an increase in plasma triglyceride or a decrease in platelet concentration.

A pharmaceutical composition for treating and/or preventing at least one of heart diseases, heart disease complications, skeletal muscle diseases, and skeletal muscle conditions, the pharmaceutical composition comprising a compound represented by Formula (I):

EP 4 434 520 A1

[Chemical Formula 1]

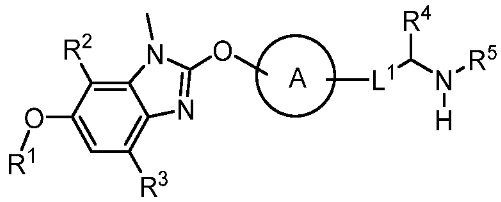

(I)

wherein $R^1$ is haloalkyl or non-aromatic carbocyclyl,
$R^2$ is a hydrogen atom or halogen,
$R^3$ is halogen,
ring A is a group represented by the formula:

[Chemical Formula 2]

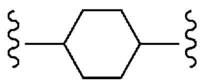
,
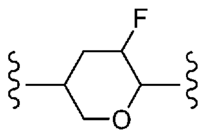
,
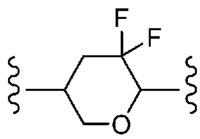
,
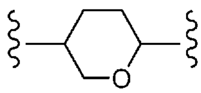
or
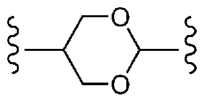

$-L^1-$ is $-O-(CH_2)-$, $-(CH_2)_2-$, or the like,
$R^4$ is alkyl or haloalkyl, and
$R^5$ is alkylcarbonyl or carbamoyl,
or a pharmaceutically acceptable salt thereof.

## Description

[TECHNICAL FIELD]

The present invention relates to a new pharmaceutical composition for treating and/or preventing at least one of heart diseases, heart disease complications, skeletal muscle diseases, and skeletal muscle conditions.

[BACKGROUND ART]

Triglyceride deposit cardiomyovasculopathy (hereinafter also referred to as TGCV) is a novel disease unit first reported in 2008, and is an incurable disease causing severe heart failure and arrhythmia as a result of accumulation of triglyceride (TG) in cardiomyocytes and coronary sclerosis foci (Non-patent Document 1). As a cause of TGCV, a defect of adipose triglyceride lipase (ATGL), which is an essential enzyme for intracellular triglyceride lipolysis, has been known so far. Long-chain fatty acids, which normally serve as energy sources in the heart, cannot be metabolized intracellularly by TGCV, and are accumulated as TG in the cardiovascular blood vessels, causing the heart to become in a state of obesity, so to speak (Non-patent Document 2). It is a condition characterized by "ectopic" accumulation of TG in cardiomyocytes and vascular smooth muscle cells, and does not necessarily correlate with body mass index (BMI), body weight, obesity, and the like, which are considered to reflect the amount of accumulated triglyceride in physiologically storing tissues. In addition, since it is based on intracellular metabolic abnormalities, it is not directly related to the serum TG value.

Although the treatment of TGCV is under research, it has recently been reported that capric acid, which is a medium chain fatty acid, has a potent intracellular TG content lowering activity, and is useful for the prevention or treatment of diabetic cardiovascular complications in which triglyceride is accumulated in myocardial or vascular tissues or cells (Patent Document 1).

However, no TGCV therapeutic agent has been put on the market so far, and the establishment of a method for treating the present disease is an important clinical issue.

ACC is an enzyme that carboxylates acetyl-CoA to convert it into malonyl-CoA, and is involved in metabolism of fatty acids. There are two isoforms of ACC: acetyl-CoA carboxylase 1 (hereinafter referred to as ACC1) and acetyl-CoA carboxylase 2 (hereinafter referred to as ACC2).

ACC2 is expressed mainly in the heart and skeletal muscles, and malonyl-CoA produced by ACC2 inhibits carnitine palmitoyltransferase I (CPT-I), thereby inhibiting oxidation of fatty acids.

In ACC2 deficient mice, continuous fatty acid oxidation occurs due to a decrease in an amount of malonyl-CoA in the heart and skeletal muscles, and a decrease in body weight is observed regardless of an increase in food intake. Furthermore, it has also been reported that ACC2 deficient mice have acquired resistance to diabetes and obesity induced by administration of a high-fat/high-carbohydrate diet.

The above findings suggest that ACC2 is involved in diseases such as diabetes and obesity, and an inhibitor thereof is an antidiabetic drug or an antiobesity drug.

On the other hand, ACC1 deficient mice are lethal in the fetal period, and therefore there is a demand for a selective inhibitor that inhibits ACC2 without inhibiting ACC1 (Non-patent Document 3).

Patent Documents 2 and 3 describe a method for treating nonalcoholic fatty liver disease using a thienopyrimidine derivative having inhibitory activity against both ACC1 and ACC2. For example, compounds shown below are known as firsocostat (an ACC1/2 dual inhibitor), and have been developed for an indication of nonalcoholic steatohepatitis and the like. Firsocostat is designed to be selectively taken up by the liver via a transporter.

[Chemical Formula 1]

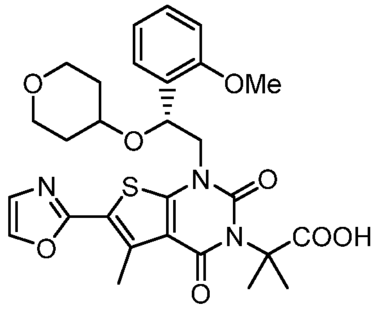

For firsocostat, top-line performance of a phase II ATLAS study over 48 weeks has been published on December

16, 2019. The phase II ATLAS study is a randomized, double-blind, placebo-controlled study to evaluate safety and efficacy of a monotherapy and a dual combination therapy with 30 mg of cilofexor (a selective nonsteroidal FXR agonist), 20 mg of firsocostat, and 18 mg of selonsertib (an ASK1 inhibitor) in patients with advanced fibrosis (F3-F4) due to NASH. As a result, it has been reported that no statistically significant increase was observed in a proportion of patients who achieved improvement in fibrosis of one or more stages without deterioration of NASH, which is a primary efficacy endpoint, in any regimen. In a previously conducted phase II study over 12 weeks, an elevated plasma triglyceride concentration has been reported in NAFLD patients receiving firsocostat. An elevated plasma triglyceride concentration has also been reported in NAFLD patients receiving another ACC1/2 dual inhibitor, MK-4074. An elevated plasma triglyceride concentration is known to increase an occurrence of cardiovascular events, and the cardiovascular events have been reported to be a most frequent cause of death in NASH patients. NAFLD is an independent risk factor for cardiovascular disease, and it has been reported that a risk of cardiovascular disease increases as a condition progresses to NASH. The ATLAS study has reported that a plasma triglyceride concentration is increased in NASH patients using firsocostat and cilofexor in combination.

Patent Document 4 describes an ACC1/2 dual inhibitor. For example, a compound shown below is known as PF-05175157 (an ACC1/2 dual inhibitor) and reduced a platelet concentration by repeated administration to healthy subjects.

[Chemical Formula 2]

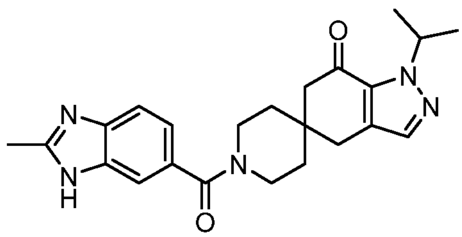

It has been reported that this is because production of platelets was reduced by suppression of fatty acid synthesis via ACC1 inhibition in the bone marrow.

Patent Documents 5 and 6 describe benzimidazole derivatives that specifically inhibit ACC2. Further, Patent Document 7 describes that a benzimidazole derivative is useful as a therapeutic agent for NASH.

In addition, Patent Documents 8 to 20 and Non-patent Documents 4 to 6 describe a compound similar to the benzimidazole derivative contained in the pharmaceutical composition of the present invention as an ACC inhibitor.

Patent Documents 21 to 26 describe that an ACC inhibitor is useful as a therapeutic agent for sarcopenia, but do not describe a benzimidazole derivative contained in the pharmaceutical composition of the present invention.

Non-patent Document 6 describes that a specific ACC2 inhibitor decreases the lipid content in skeletal muscle cells, and improves insulin resistance and hyperglycemia. Further, a benzimidazole derivative contained in the pharmaceutical composition of the present invention is not described.

[PRIOR ART REFERENCES]

[Patent Document]

[Patent Document 1] International Publication WO 2013/031729 A
[Patent Document 2] International Publication WO 2013/071169 A
[Patent Document 3] International Publication WO 2016/112305 A
[Patent Document 4] International Publication WO 2011/058474 A
[Patent Document 5] International Publication WO 2015/056782 A
[Patent Document 6] International Publication WO 2016/159082 A
[Patent Document 7] International Publication WO 2021/235508 A
[Patent Document 8] International Publication WO 2018/007430 A
[Patent Document 9] International Publication WO 2015/036892 A

[Patent Document 10] International Publication WO 2014/061693 A
[Patent Document 11] International Publication WO 2014/056771 A
[Patent Document 12] International Publication WO 2013/092616 A
[Patent Document 13] International Publication WO 2008/079610 A
[Patent Document 14] International Publication WO 2013/035827 A
[Patent Document 15] International Publication WO 2010/003624 A
[Patent Document 16] International Publication WO 2007/095601 A
[Patent Document 17] Specification of US Patent Application Publication No. 20060178400
[Patent Document 18] International Publication WO 2011/136385 A
[Patent Document 19] International Publication WO 2012/074126 A
[Patent Document 20] International Publication WO 2016/041845 A
[Patent Document 21] International Publication WO 2007/119833 A
[Patent Document 22] International Publication WO 2008/090944 A
[Patent Document 23] International Publication WO 2008/102749 A
[Patent Document 24] JP 2009-196966 A
[Patent Document 25] International Publication WO 2010/050445 A
[Patent Document 26] International Publication WO 2011/136385 A

[Non-patent Document]

[Non-patent Document 1] N. Engl. J. Med., 359(22), 2396-2398, 2008
[Non-patent Document 2] J. Atheroscler. Thromb., 16(5), 702-705, 2009
[Non-patent Document 3] PNAS August 23, 2005 102 (34) 12011-12016
[Non-patent Document 4] Molecular Diversity (2013), 17(1), 139-149
[Non-patent Document 5] Journal of Medicinal Chemistry (2010), 53(24), 8679-8687
[Non-patent Document 6] J Pharmacol Exp Ther 372: 256-263, 2020

[SUMMARY OF THE INVENTION]

[PROBLEMS TO BE SOLVED BY THE INVENTION]

An object of the present invention is to provide a pharmaceutical composition for treating and/or preventing at least one of heart diseases, heart disease complications, skeletal muscle diseases, and skeletal muscle conditions, the pharmaceutical composition having an excellent ACC2-selective inhibitory effect and having no side effects such as an increase in plasma triglyceride or a decrease in platelet concentration. More preferably, an object of the present invention is to provide a pharmaceutical composition for treating and/or preventing triglyceride deposit cardiomyovasculopathy or a skeletal muscle disease or condition caused by the accumulation of triglyceride in skeletal muscle cells.

[MEANS FOR SOLVING THE PROBLEM]

As a result of repeated studies to solve the above problems, the present inventors have found that among the compounds having an ACC2-selective inhibitory effect described in Patent Documents 5 and 6, a specific compound (compound having high ACC2 selectivity and excellent metabolic stability) is effective for treating and/or preventing at least one of heart diseases, heart disease complications, skeletal muscle diseases, and skeletal muscle conditions, and has no side effects such as an increase in plasma triglyceride or a decrease in platelet concentration, thereby completing the present invention. An ACC2-selective inhibitor contained in the pharmaceutical composition of the present invention can avoid side effects due to ACC1 inhibition and inhibit systemic ACC2. As a result, unlike an ACC1/2 dual inhibitor and a liver-selective ACC1/2 dual inhibitor such as firsocostat, the ACC2-selective inhibitor in the pharmaceutical composition of the present invention does not cause a decrease in platelet and does not increase a plasma triglyceride concentration, and can exert a metabolic improvement action based on systemic ACC2 inhibition. The present invention relates to the following.

[1] A pharmaceutical composition for treating and/or preventing at least one of heart diseases and heart disease complications, the pharmaceutical composition comprising a compound (the compound may also be referred to as "compound of the present invention" in the present specification) represented by Formula (I):

[Chemical Formula 3]

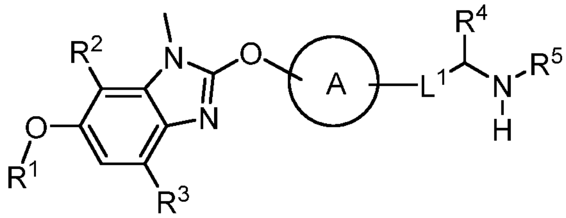

(I)

wherein $R^1$ is haloalkyl or non-aromatic carbocyclyl,
$R^2$ is a hydrogen atom or halogen,
$R^3$ is halogen,
ring A is a group represented by the formula:

[Chemical Formula 4]

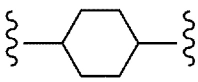 , 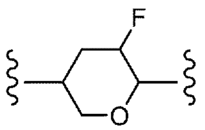 , 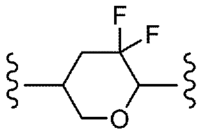 , 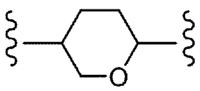 or 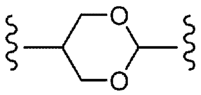

$-L^1-$ is $-O-(CH_2)-$, $-(CH_2)_2-$, $-(CH_2)-(CF_2)-$, or $-(CF_2)-(CH_2)-$ (wherein a left bond is attached to the ring A and a right bond is attached to a group represented by the formula:

[Chemical Formula 5]

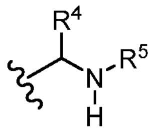

),

$R^4$ is alkyl or haloalkyl, and
$R^5$ is alkylcarbonyl or carbamoyl,
or a pharmaceutically acceptable salt thereof.

[2] The pharmaceutical composition according to the above item [1], wherein $R^1$ is non-aromatic carbocyclyl.
[3] The pharmaceutical composition according to the above item [1] or [2], wherein $R^2$ is a hydrogen atom.
[4] The pharmaceutical composition according to any one of the above items [1] to [3],
wherein the ring A is a group represented by the formula:

[Chemical Formula 6]

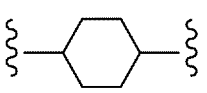 or 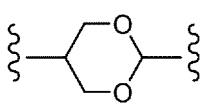

.

[5] The pharmaceutical composition according to any one of the above items [1] to [4], wherein $-L^1-$ is $-O-(CH_2)-$ or $-(CH_2)_2-$.
[6] The pharmaceutical composition according to any one of the above items [1] to [5], wherein $R^4$ is alkyl.
[7] The pharmaceutical composition according to any one of the above items [1] to [6], wherein $R^5$ is methylcarbonyl or carbamoyl.
[8] The pharmaceutical composition according to the above item [1],

wherein the compound represented by Formula (I) or the pharmaceutically acceptable salt thereof is a compound selected from the group consisting of the following formulae:

[Chemical Formula 7]

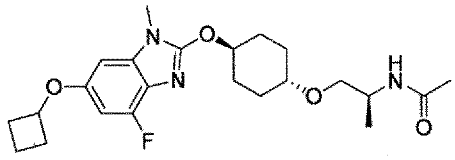,
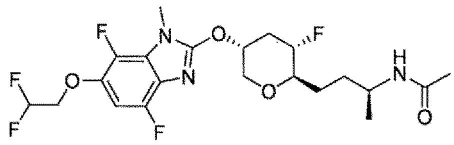,
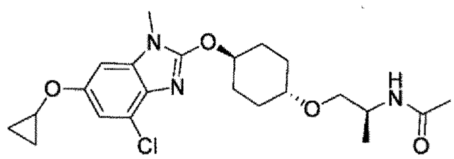,
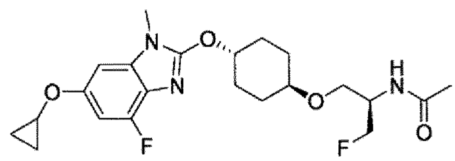,
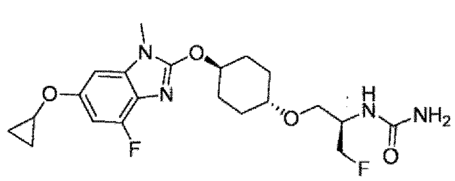,
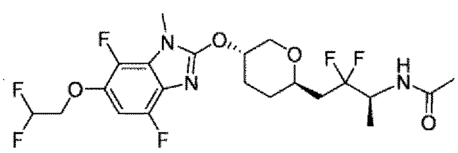,
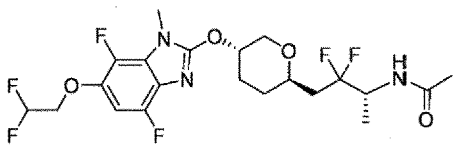,
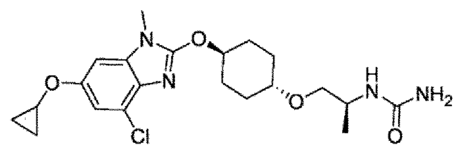,
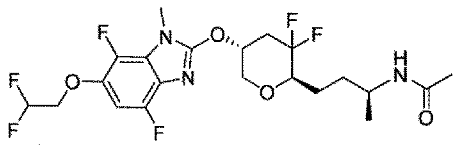,
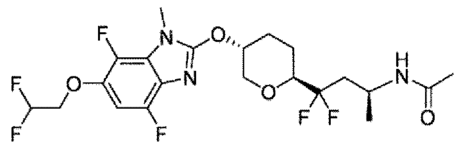,
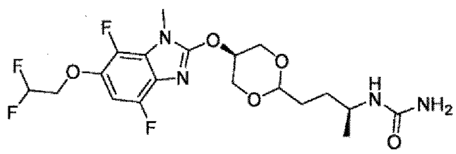,
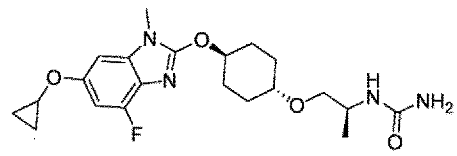,
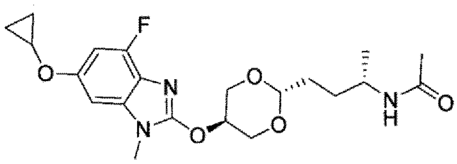,
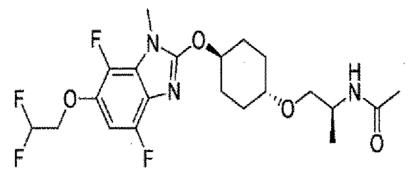

and 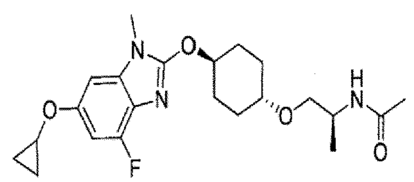

or a pharmaceutically acceptable salt thereof.

[9] The pharmaceutical composition according to any one of the above items [1] to [8], wherein the heart disease and heart disease complication are at least one disease selected from the group consisting of heart failure, angina pectoris, myocardial infarction, cardiomyopathy, arrhythmia, coronary artery disease, and skeletal muscle myopathy.
[10] The pharmaceutical composition containing the compound according to any one of the above items [1] to [8] for treating and/or preventing a disease caused by accumulation of triglyceride in at least one of cardiomyocytes, vascular smooth muscle cells, endothelial cells, skeletal muscle cells, polymorphonuclear leukocytes, renal tubular epithelial cells, and pancreatic islet cells.

[11] The pharmaceutical composition according to the above item [10], wherein the disease is at least one disease selected from the group consisting of heart failure, angina pectoris, myocardial infarction, cardiomyopathy, arrhythmia, coronary artery disease, skeletal muscle myopathy, chronic kidney disease, and impaired glucose tolerance.
[12] The pharmaceutical composition according to any one of the above items [1] to [11], administration of which causes no side effects of an increase in plasma triglyceride.
[13] The pharmaceutical composition according to any one of the above items [1] to [11], administration of which causes no side effects of a decrease in platelet concentration.
[14] A pharmaceutical composition for treating and/or preventing a skeletal muscle disease or condition, the pharmaceutical composition comprising a compound represented by Formula (I):

[Chemical Formula 8]

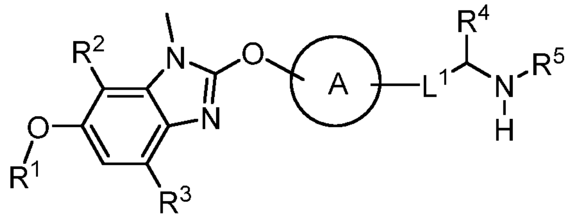
(I)

wherein $R^1$ is haloalkyl or non-aromatic carbocyclyl,
$R^2$ is a hydrogen atom or halogen,
$R^3$ is halogen,
ring A is a group represented by the formula:

[Chemical Formula 9]

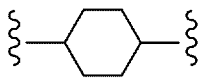 , 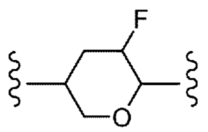 , 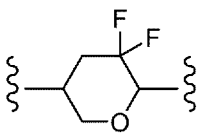 , 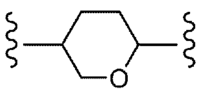 or 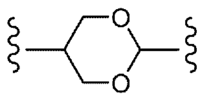

$-L^1-$ is $-O-(CH_2)-$, $-(CH_2)_2-$, $-(CH_2)-(CF_2)-$, or $-(CF_2)-(CH_2)-$ (wherein a left bond is attached to the ring A and a right bond is attached to a group represented by the formula:

[Chemical Formula 10]

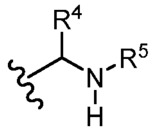 ),

$R^4$ is alkyl or haloalkyl, and
$R^5$ is alkylcarbonyl or carbamoyl,
or a pharmaceutically acceptable salt thereof.

[15] The pharmaceutical composition according to the above item [14], wherein $R^1$ is non-aromatic carbocyclyl.
[16] The pharmaceutical composition according to the above item [14] or [15], wherein $R^2$ is a hydrogen atom.
[17] The pharmaceutical composition according to any one of the above items [14] to [16],
wherein the ring A is a group represented by the formula:

[Chemical Formula 11]

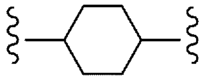

or

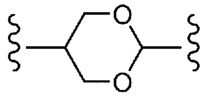

.

[18] The pharmaceutical composition according to any one of the above items [14] to [17], wherein $-L^1-$ is $-O-(CH_2)-$ or $-(CH_2)_2-$.

[19] The pharmaceutical composition according to any one of the above items [14] to [18], wherein $R^4$ is alkyl.

[20] The pharmaceutical composition according to any one of the above items [14] to [19], wherein $R^5$ is methylcarbonyl or carbamoyl.

[21] The pharmaceutical composition according to the above item [14],

wherein the compound represented by Formula (I) or the pharmaceutically acceptable salt thereof is a compound selected from the group consisting of the following formulae:

[Chemical Formula 12]

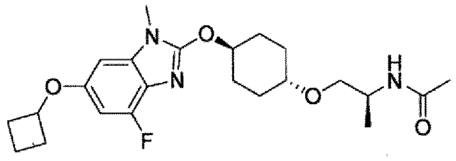 , 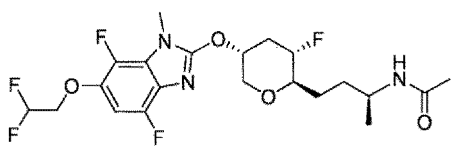 ,

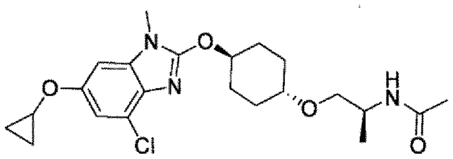 , 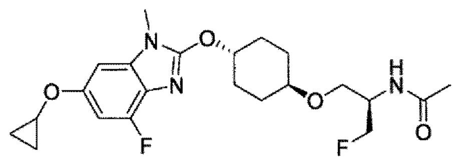 ,

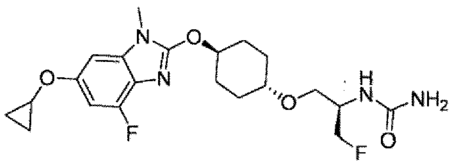 , 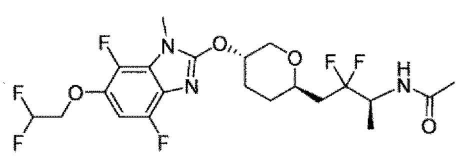 ,

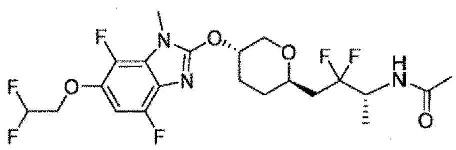 , 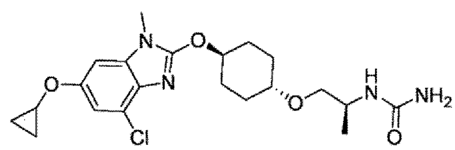 ,

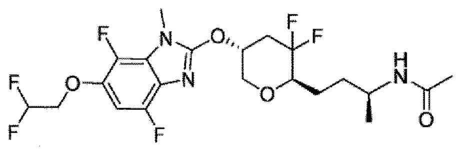 , 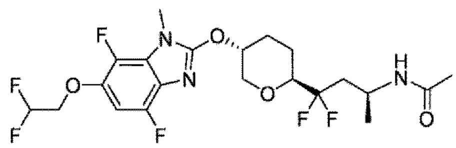 ,

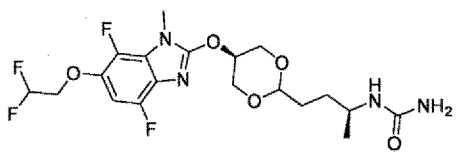 , 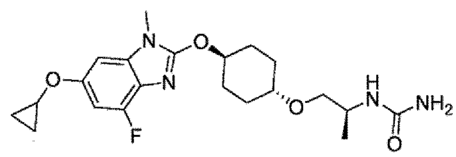 ,

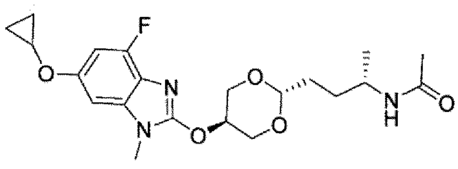 , 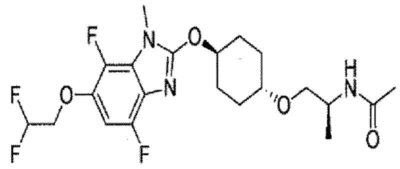

and 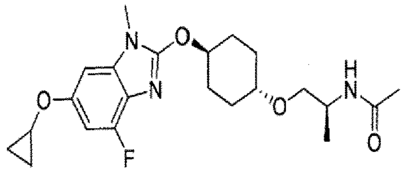

or a pharmaceutically acceptable salt thereof.

[22] The pharmaceutical composition according to any one of the above items [14] to [21], wherein the skeletal muscle disease or condition is caused by accumulation of triglyceride in skeletal muscle cells (for example, myosteatosis).

[23] The pharmaceutical composition according to any one of the above items [14] to [22], wherein the skeletal muscle disease or condition is skeletal muscle myopathy or sarcopenia.

[24] The pharmaceutical composition according to any one of the above items [14] to [23], wherein a pathology and/or symptom of the skeletal muscle disease or condition is due to a contribution of ACC2 activity.

[25] The pharmaceutical composition according to the above item [24], wherein the ACC2 activity contributes to a condition selected from the group consisting of diabetes, obesity and liver cirrhosis.

[26] The pharmaceutical composition according to any one of the above items [14] to [25], wherein the skeletal muscle disease or condition is associated with a condition selected from the group consisting of diabetes, obesity, and liver cirrhosis.

[27] The pharmaceutical composition according to the above item [26], wherein the skeletal muscle disease or condition is associated with liver cirrhosis.

[28] The pharmaceutical composition according to any one of the above items [14] to [27], wherein the pharmaceutical composition maintains or increases skeletal muscle function in a subject having the skeletal muscle disease or condition.

[29] The pharmaceutical composition according to any one of the above items [14] to [27], wherein the pharmaceutical composition promotes skeletal muscle recovery in a subject having the skeletal muscle disease or condition.

[30] The pharmaceutical composition according to any of the above items [14] to [27], wherein the pharmaceutical composition maintains or increases skeletal muscle function in a subject.

[EFFECT OF THE INVENTION]

The pharmaceutical composition of the present invention containing the compound represented by Formula (I) or the pharmaceutically acceptable salt thereof has an excellent effect of being effective for treating and/or preventing at least one of heart diseases, heart disease complications, skeletal muscle diseases, and skeletal muscle conditions, particularly triglyceride deposit cardiomyovasculopathy or a skeletal muscle disease or condition caused by the accumulation of triglyceride in skeletal muscle cells. In addition, the pharmaceutical composition has high safety without side effects such as an increase in plasma triglyceride or a decrease in platelet concentration.

[BRIEF DESCRIPTION OF DRAWINGS]

Fig. 1 shows the metabolism of long-chain fatty acids in the myocardium when Compound 1-12 and Compound 1-13 are administered to Atgl knockout mice.

Fig. 2 shows a plasma triglyceride concentration when Compound 1-12 was administered to mice fed an ultra-high fat, choline-deficient, methionine-lowered diet.

Fig. 3 shows a plasma triglyceride concentration when Compound 1-13 was administered to mice fed an ultra-high fat, choline-deficient, methionine-lowered diet.

Fig. 4 shows the lipid content in skeletal muscle cells when Compound 1-13 was administered to SD rats.

Fig. 5 shows the lipid content in skeletal muscle cells when Compound 1-12 was administered to leptin receptor-deficient mice.

Fig. 6 shows the lipid content in skeletal muscle cells when Compound 1-12 was administered to diabetic model rats.

Fig. 7 shows the lipid content in skeletal muscle cells when Compound 1-14 was administered to high-fat-diet-challenged mice.

Fig. 8 shows the lipid content in skeletal muscle cells and the lipid content outside skeletal muscle cells when wild-type mice and ACC2 knockout mice were challenged with a high-fat diet.

Fig. 9 shows the spontaneous motility when Compound 1-13 was administered to Atgl knockout mice.

Fig. 10 shows the spontaneous motility in Atgl knockout mice and Atgl-ACC2 double knockout mice.

[MODE FOR CARRYING OUT THE INVENTION]

The meaning of each term used in the present specification will be described below. Unless otherwise specified, each term is used in the same meaning when used alone or in combination with another term.

The term "consisting of" means having only components.

The term "comprising" means being not limited to the components, but not excluding elements that are not described.

Hereinafter, the present invention will be described with reference to embodiments. Throughout the present specification, an expression in a singular form should be understood as also including the concept of its plural form, unless otherwise stated. Therefore, singular articles (for example, "a", "an", "the", and the like in English) should be understood as also including the concept of their plural form, unless otherwise stated.

In addition, the terms used in the present specification should be understood as being used in the meanings commonly used in the art unless otherwise stated. Therefore, unless otherwise defined, all terminology and scientific terms used in the present specification have the same meanings as commonly understood by those skilled in the art to which the present invention belongs. If there is a contradiction, the present specification (including definitions) precedes.

The term "halogen" includes a fluorine atom, a chlorine atom, a bromine atom, and an iodine atom. In particular, a fluorine atom and a chlorine atom are preferable.

The term "alkyl" includes a linear or branched hydrocarbon group having 1 to 15 carbon atoms, preferably 1 to 10 carbon atoms, more preferably 1 to 6 carbon atoms, and still more preferably 1 to 4 carbon atoms. Examples thereof include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

A preferred embodiment of "alkyl" includes methyl, ethyl, n-propyl, isopropyl, n-butyl, isobutyl, sec-butyl, tert-butyl, and n-pentyl. A more preferred embodiment includes methyl, ethyl, n-propyl, isopropyl, and tert-butyl. A particularly preferred embodiment includes methyl.

The term "haloalkyl" means a group wherein one or more arbitrary hydrogen atoms of the above "alkyl" are substituted with the above "halogen". Examples thereof include monofluoromethyl, monofluoroethyl, monofluoropropyl, 2,2-difluoroethyl, 2,2,3,3,3-pentafluoropropyl, monochloromethyl, trifluoromethyl, trichloromethyl, 2,2,2-trifluoroethyl, 2,2,2-trichloroethyl, 1,2-dibromoethyl, and 1,1,1-trifluoropropan-2-yl.

The term "alkylcarbonyl" means a group wherein the above "alkyl" is bonded to a carbonyl group. Examples thereof include methylcarbonyl, ethylcarbonyl, propylcarbonyl, isopropylcarbonyl, tert-butylcarbonyl, isobutylcarbonyl, sec-butylcarbonyl, penthylcarbonyl, isopenthylcarbonyl, and hexylcarbonyl. A preferred embodiment of "alkylcarbonyl" includes methylcarbonyl, ethylcarbonyl, n-propylcarbonyl, and the like. A more preferred embodiment includes methylcarbonyl.

The term "aromatic carbocyclyl" means a cyclic aromatic hydrocarbon group that is monocyclic or bicyclic or more. Examples thereof include phenyl, naphthyl, anthryl, and phenanthryl.

A preferred embodiment of "aromatic carbocyclyl" includes phenyl.

The term "aromatic carbocycle" means a ring derived from the above "aromatic carbocyclyl".

The term "non-aromatic carbocyclyl" means a cyclic saturated hydrocarbon group or a cyclic unsaturated non-aromatic hydrocarbon group that is monocyclic or bicyclic or more. The "non-aromatic carbocyclyl" that is bicyclic or more also includes non-aromatic carbocyclyl that is monocyclic or bicyclic or more fused with a ring in the above "aromatic carbocyclyl".

Furthermore, the "non-aromatic carbocyclyl" also includes a cross-linked group or a group forming a spiro ring, as shown below.

[Chemical Formula 13]

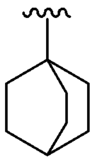

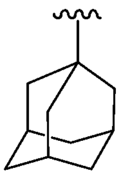

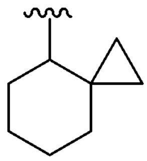

The non-aromatic carbocyclyl that is monocyclic has preferably 3 to 16 carbon atoms, more preferably 3 to 12 carbon atoms, further preferably 3 to 6 carbon atoms, and particularly preferably 3 to 4 carbon atoms. Examples thereof include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, and cyclohexadienyl.

The non-aromatic carbocyclyl that is bicyclic or more has preferably 8 to 20 carbon atoms, and more preferably 8 to 16 carbon atoms. Examples thereof include indanyl, indenyl, acenaphthyl, tetrahydronaphthyl, and fluorenyl.

The term "non-aromatic carbocycle" means a ring derived from the above "non-aromatic carbocyclyl".

The term "aromatic heterocyclyl" means an aromatic cyclic group that is monocyclic or bicyclic or more, having one or more same or different heteroatoms selected optionally from O, S, and N in the ring(s).

The aromatic heterocyclyl that is bicyclic or more also includes an aromatic heterocyclyl that is monocyclic or bicyclic or more fused with a ring in the above "aromatic carbocyclyl". The bond may be present on any of the rings.

The aromatic heterocyclyl that is monocyclic is preferably a 5- to 8-membered group, and more preferably a 5- or 6-membered group. Examples of the 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of 6-membered aromatic heterocyclyl include pyridyl, pyridazinyl, pyrimidinyl, pyrazinyl, and triazinyl.

The aromatic heterocyclyl that is bicyclic is preferably an 8- to 10-membered group, and more preferably a 9- or 10-membered group. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl.

The aromatic heterocyclyl that is tricyclic or more is preferably a 13- to 15-membered group. Examples thereof

include carbazolyl, acridinyl, xanthenyl, phenothiazinyl, phenoxathiinyl, phenoxazinyl, and dibenzofuryl.

The term "aromatic heterocycle" means a ring derived from the above "aromatic heterocyclyl".

The term "non-aromatic heterocyclyl" means a non-aromatic cyclic group that is monocyclic or bicyclic or more, having one or more same or different heteroatoms selected optionally from O, S, and N in the ring(s). The non-aromatic heterocyclyl that is bicyclic or more also includes a non-aromatic heterocyclyl that is monocyclic or bicyclic or more fused with each ring in the "aromatic carbocyclyl", the "non-aromatic carbocyclyl", and/or the "aromatic heterocyclyl" described above, and further non-aromatic carbocyclyl that is monocyclic or bicyclic or more fused with a ring in the above "aromatic heterocyclyl". The bond may be present on any of the rings.

Furthermore, the "non-aromatic heterocyclyl" also includes a cross-linked group or a group forming a spiro ring, as shown below.

[Chemical Formula 14]

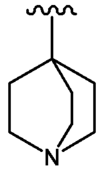

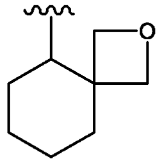

The non-aromatic heterocyclyl that is monocyclic is preferably a 3- to 8-membered group, and more preferably a 5- or 6-membered group.

Examples of the 3-membered non-aromatic heterocyclyl include thiiranyl, oxiranyl, and aziridinyl. Examples of the 4-membered non-aromatic heterocyclyl include oxetanyl and azetidinyl. Examples of the 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, and thiolanyl. Examples of the 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydrooxazinyl, tetrahydropyridazinyl, hexahydropyrimidinyl, dioxazinyl, thiinyl, and thiazinyl. Examples of the 7-membered non-aromatic heterocyclyl include hexahydroazepinyl, tetrahydrodiazepinyl, and oxepanyl.

The non-aromatic heterocyclyl that is bicyclic or more is preferably an 8- to 20-membered group, and more preferably an 8- to 10-membered group. Examples thereof include indolinyl, isoindolinyl, chromanyl, and isochromanyl.

The term "non-aromatic heterocycle" means a ring derived from the above "non-aromatic heterocyclyl".

The term "disease" means a disease/disorder/syndrome/symptom at various stages from pre-onset to advanced stages (including symptoms and signs of the disease, including presymptomatic and other signs of the disease).

The term "condition" means a state of low performance relative to an individual's potential and goals.

The term "increasing skeletal muscle function" means improving the skeletal muscle function of an individual. The term "pharmaceutical composition that increases skeletal muscle function" also encompasses the prophylactic use (application) of the pharmaceutical composition.

The term "promoting skeletal muscle recovery" means improving skeletal muscle function in an individual having a skeletal muscle disease or condition.

(Use of Pharmaceutical Composition of Present Invention for Heart Disease and Heart Disease Complications)

Since the compound of the present invention has a metabolic effect of fatty acid on the myocardium, the heart disease or the heart disease complication is not particularly limited as long as it is a disease in which triglyceride is accumulated in tissues or cells of the myocardium and blood vessels of the heart or a disease caused by accumulation of triglyceride, but it is preferably at least one disease selected from the group consisting of a disease in which a blood vessel is narrowed or occluded due to accumulation of triglyceride in a blood vessel, a disease caused by the narrowing or occlusion, and a disease caused by accumulation of triglyceride in the myocardium. For example, clinically, the heart disease or the heart disease complication is preferably a disease in which at least one selected from the group consisting of myocardial hypertrophy, diffuse stenosis and afferent stenosis of coronary arteries is observed.

Therefore, the "heart disease and heart disease complication" of the present invention is, for example, at least one disease selected from the group consisting of heart failure, angina pectoris, myocardial infarction, cardiomyopathy, arrhythmia, and coronary artery disease.

Furthermore, the compound of the present invention is also effective for a disease caused by accumulation of triglyceride in at least one of cardiomyocytes, vascular smooth muscle cells, endothelial cells, skeletal muscle cells, polymorphonuclear leukocytes, renal tubular epithelial cells, and pancreatic islet cells, that is, "triglyceride deposit cardiomyovasculopathy (TGCV)". The "triglyceride deposit cardiomyovasculopathy" is, for example, at least one disease

selected from the group consisting of heart failure, angina pectoris, myocardial infarction, cardiomyopathy, arrhythmia, coronary artery disease, skeletal muscle myopathy, chronic kidney disease, and impaired glucose tolerance.

The compound of the present invention is also effective for atherosclerosis caused by accumulation of triglyceride in blood vessels. In particular, the compound also has a therapeutic/preventive effect even in a case in which the symptoms are not improved by the cholesterol lowering agent.

(Use of pharmaceutical composition of present invention for skeletal muscle disease or condition)

The pharmaceutical composition of the present invention contains a compound for treating and/or preventing a skeletal muscle disease or condition. The compound or a pharmaceutically acceptable salt thereof is represented by Formula (I).

In certain embodiments, a "skeletal muscle disease or condition" is caused by the accumulation of triglyceride in skeletal muscle cells. In some embodiments, the "skeletal muscle disease or condition" is myosteatosis. Myosteatosis means a change in muscle quality due to the accumulation of triglyceride in skeletal muscle cells. In some embodiments, the "skeletal muscle disease or condition" may be associated with a liver disease. Non-limiting examples of such a liver disease include progressive or late stage liver diseases.

In certain embodiments, the "skeletal muscle disease or condition" is a skeletal muscle myopathy. Skeletal muscle myopathy is characterized by progressive degeneration of muscle tissues by abnormal or insufficient structural support proteins present. In some embodiments, the skeletal muscle myopathy may be associated with disintegration of muscle fibers and/or loss of muscle strength. In some embodiments, the skeletal muscle myopathy may be hereditary or acquired myopathy. In some embodiments, the skeletal muscle myopathy may be a congenital myopathy, a mitochondrial myopathy, a metabolic myopathy, a muscular dystrophy, a toxic myopathy, an endocrine myopathy, an infectious myopathy, a severe disease myopathy, a myopathy caused by an electrolyte imbalance, and any combination thereof.

In certain embodiments, the "skeletal muscle disease or condition" is sarcopenia. Sarcopenia is a type of muscle atrophy that includes a decrease in the size and number of muscle fibers. In some embodiments, sarcopenia may be associated with a loss of one or more of muscle strength, muscle mass, and muscle function.

In certain embodiments, the pathology and/or symptom of the "skeletal muscle disease or condition" is due to a contribution of ACC2 activity. In some embodiments, ACC2 activity is associated with the accumulation of triglyceride in skeletal muscle cells. In certain embodiments, ACC2 activity contributes to a condition selected from the group consisting of myosteatosis, skeletal muscle myopathy, sarcopenia, and any combination thereof.

In certain embodiments, ACC2 activity contributes to a condition selected from the group consisting of diabetes, obesity, and liver cirrhosis. In certain embodiments, ACC2 activity concurrently contributes to a combination of two or more conditions selected from the group consisting of diabetes, obesity, liver cirrhosis, myosteatosis, skeletal muscle myopathy, and sarcopenia.

In certain embodiments, "skeletal muscle disease or condition" is associated with a condition selected from the group consisting of diabetes, obesity, and liver cirrhosis. In certain embodiments, myosteatosis is concurrently associated with diabetes, obesity, or liver cirrhosis. In certain embodiments, skeletal muscle myopathy is concurrently associated with diabetes, obesity, or liver cirrhosis. In certain embodiments, sarcopenia is concurrently associated with diabetes, obesity, or liver cirrhosis.

In certain embodiments, the "skeletal muscle disease or condition" is associated with liver cirrhosis. In certain embodiments, myosteatosis is associated with liver cirrhosis. In certain embodiments, skeletal muscle myopathy is associated with liver cirrhosis. In certain embodiments, sarcopenia is associated with liver cirrhosis.

In certain embodiments, the pharmaceutical composition of the present invention maintains or increases skeletal muscle function in a subject. In certain embodiments, the pharmaceutical composition of the present invention promotes skeletal muscle recovery in a subject having a skeletal muscle disease or condition.

In certain embodiments, "skeletal muscle function" and "skeletal muscle recovery" can be determined using manual muscle testing. This determination includes measuring muscle performance against resistance and evaluating its performance on a scale of 0 to 5. In certain embodiments, muscle performance is measured by whether or not the following conditions apply: (1) the muscle can move only slightly like a cramp, and cannot be moved in a full range of motion; (2) the muscle can be moved in a full range of motion under zero gravity; (3) the muscle can be moved in a full range of motion under normal gravity; (4) the muscle can be moved in a full range of motion under the presence of some resistance; and (5) the muscle can be moved in a full range of motion under the presence of strong resistance. In certain embodiments, the measurable muscles include shoulder abductors, elbow flexors, elbow extensors, wrist extensors, digital flexors, hand intrinsic muscles, hip flexors, knee extensors, dorsal flexors, thumb extensors, and plantar flexors.

In certain embodiments, "skeletal muscle function" and "skeletal muscle recovery" can be determined using standard tests including hand grip test, chair stand test, walking speed test, short physical performance battery (SPPB), and timed up and go test (TUG). In certain embodiments, skeletal muscle function and skeletal muscle recovery can be

determined using imaging studies including dual-energy x-ray absorptiometry (DEXA or DXA) and bioelectrical impedance analysis (BIA). In certain embodiments, skeletal muscle function and skeletal muscle recovery can be determined using tests including blood tests, electromyography (EMG), magnetic resonance imaging (MRI), genetic testing, and muscle biopsy.

In certain embodiments, "skeletal muscle function" and "skeletal muscle recovery" can be determined using a frailty index. For example, the liver weakness index (LFI) can be used. The LFI includes three tests based on performance (grip strength, chair stand, balance). For example, grip strength is measured in kilograms using a handheld dynamometer on the dominant hand of the subject. The average of results of three trials is calculated for analysis. For example, in a timed chair stand, the number of seconds it takes for a subject to complete five standups from a chair with arms crossed over the chest is measured. For example, in the balance test, the number of seconds in which the subject can maintain balance is measured assuming that the subject has three postures (with legs arranged side by side, in semi-tandem, and vertically (tandem)) and each posture has a maximum of 10 seconds. Any one or more of the three factors can be used for evaluation.

(Compound Represented by Formula (I) Contained in Pharmaceutical Composition of Present Invention)

Preferred embodiments of $R^1$, $R^2$, $R^3$, $R^4$, $R^5$, $L^1$, and ring A in the compound represented by Formula (I) are described below. Examples of the compound represented by Formula (I) include embodiments of all combinations of specific examples described below.

$R^1$ is haloalkyl or non-aromatic carbocyclyl.
$R^1$ is preferably non-aromatic carbocyclyl.
$R^1$ is more preferably non-aromatic carbocyclyl that is monocyclic, and particularly preferably cyclopropyl or cyclobutyl.

Another preferred embodiment of $R^1$ includes preferably 2,2-difluoroethyl.

$R^2$ is a hydrogen atom or halogen.
$R^2$ is preferably a hydrogen atom or a fluorine atom.
$R^2$ is more preferably a hydrogen atom.
$R^3$ is halogen.
$R^3$ is preferably a fluorine atom or a chlorine atom.
$R^3$ is more preferably a fluorine atom.
$R^4$ is alkyl or haloalkyl.
$R^4$ is preferably alkyl.
$R^4$ is more preferably methyl.

Another preferred embodiment of $R^4$ includes preferably monofluoromethyl.

$R^5$ is alkylcarbonyl or carbamoyl.
$R^5$ is preferably methylcarbonyl or carbamoyl.

The ring A is a group represented by the formula:

[Chemical Formula 15]

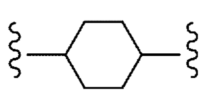 , 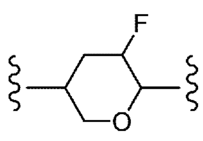 , 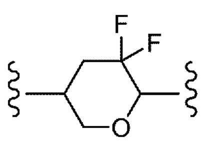 , 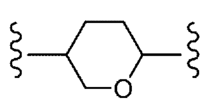 or 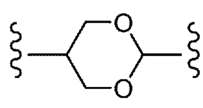

The ring A is preferably a group represented by the formula:

[Chemical Formula 16]

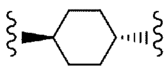,
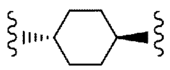,
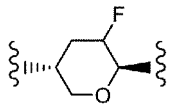,
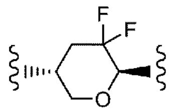,
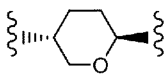
or
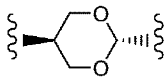

The ring A is more preferably a group represented by the formula:

[Chemical Formula 17]

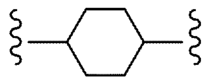
or
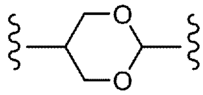

The ring A is further preferably a group represented by the formula:

[Chemical Formula 18]

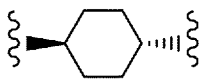
or
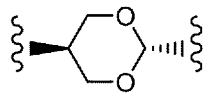

$-L^1-$ is $-O-(CH_2)-$, $-(CH_2)_2-$, $-(CH_2)-(CF_2)-$, or $-(CF_2)-(CH_2)-$ wherein a left bond is attached to the ring A and a right bond is attached to a group represented by the formula:

[Chemical Formula 19]

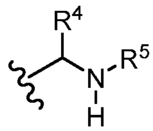

$-L^1-$ is preferably $-O-(CH_2)-$ or $-(CH_2)_2-$.

The compound represented by Formula (I) is particularly preferably a compound shown below or a pharmaceutically acceptable salt thereof.

[Chemical Formula 20]

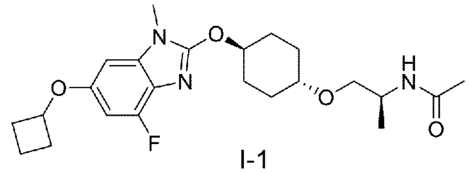
I-1
,

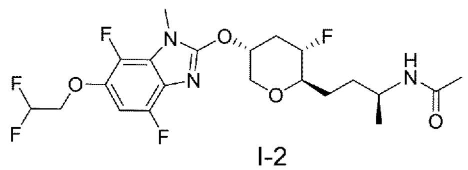
I-2
,

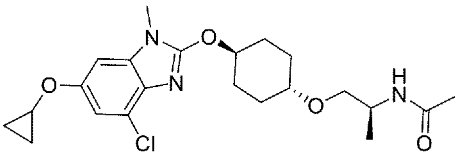
I-3
,

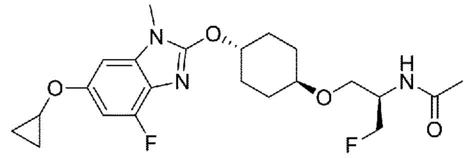
I-4
,

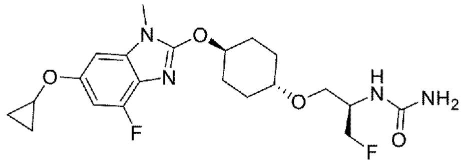
I-5
,

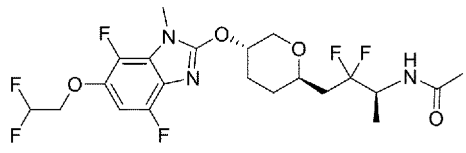
I-6
,

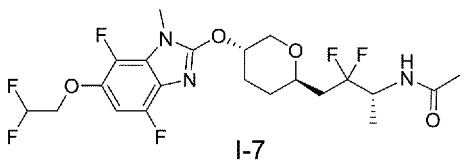
I-7
,

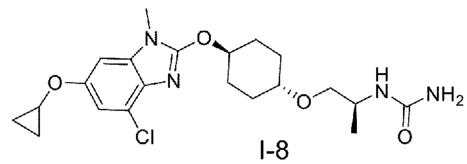
I-8
,

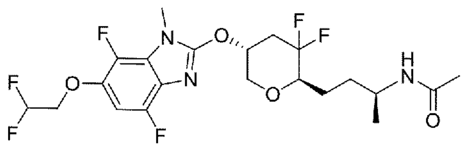
I-9
,

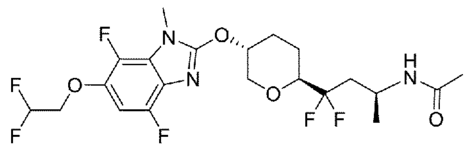
I-10
,

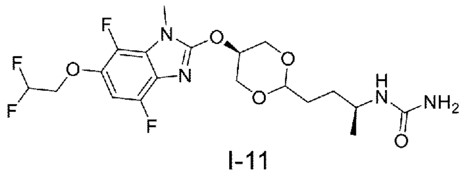
I-11
,

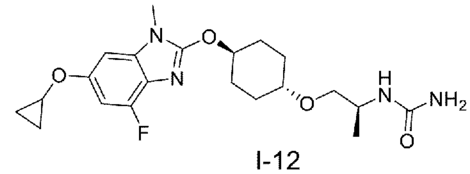
I-12
,

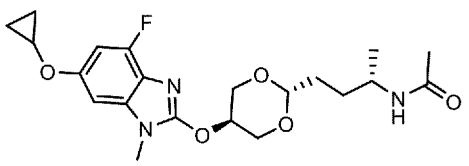
I-13
,

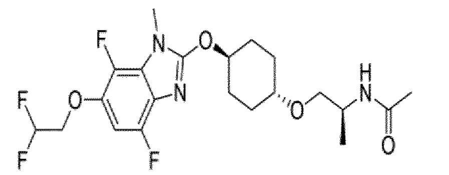
I-14
,

or

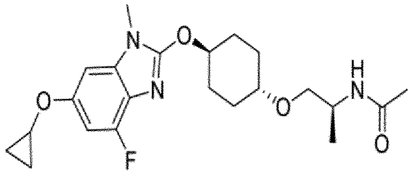
I-15

The pharmaceutical composition of the present invention for treating and/or preventing at least one of heart

diseases, heart disease complications, skeletal muscle diseases, and skeletal muscle conditions is characterized by being a pharmaceutical composition comprising, as an active ingredient, a compound represented by Formula (I):

[Chemical Formula 21]

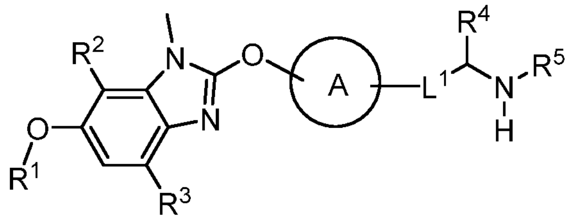

(I)

wherein each symbol has the same meaning as described above, or a pharmaceutically acceptable salt thereof.

The compound represented by Formula (I) can be synthesized according to a known method, for example, the methods described in International Publication WO 2015/056782 A and International Publication WO 2016/159082 A.

The compound represented by Formula (I) is not limited to specific isomers, but includes all possible isomers (e.g., keto-enol isomers, imine-enamine isomers, diastereoisomers, optical isomers, rotamers, or the like), racemates, or mixtures thereof.

One or more hydrogen, carbon, and/or other atoms in the compound represented by Formula (I) may be substituted with isotopes of hydrogen, carbon, and/or other atoms, respectively. Examples of such an isotope include hydrogen, carbon, nitrogen, oxygen, phosphorus, sulfur, fluorine, iodine, and chlorine such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$, and $^{36}Cl$. The compound represented by Formula (I) also includes a compound substituted with such an isotope. The compound substituted with the isotope is also useful as a pharmaceutical product and includes all radiolabeled forms of the compound represented by Formula (I). Furthermore, a "radiolabeling method" for production of the "radiolabeled form" is also included in the present invention, and the "radiolabeled form" is useful as a tool for metabolic pharmacokinetic studies and for research and/or diagnosis in binding assay.

The radiolabeled form of the compound represented by Formula (I) can be prepared by a method well known in the art. For example, a tritium-labeled compound represented by Formula (I) can be prepared by introducing tritium into a specific compound represented by Formula (I) by catalytic dehalogenation reaction using tritium. This method includes reacting an appropriately halogenated precursor of the compound represented by Formula (I) with tritium gas in the presence of an appropriate catalyst, such as Pd/C, and in the presence or absence of a base. Regarding other appropriate methods for preparing a tritium-labeled compound, "Isotopes in the Physical and Biomedical Sciences, Vol. 1, Labeled Compounds (Part A), Chapter 6 (1987)" can be referred to. A $^{14}C$-labeled compound can be prepared by using a raw material having $^{14}C$ carbon.

Examples of the pharmaceutically acceptable salt of the compound represented by Formula (I) include salts of the compound represented by Formula (I) with alkali metals (e.g., lithium, sodium, potassium, and the like), alkaline earth metals (e.g., calcium, barium, and the like), magnesium, transition metals (e.g., zinc, iron, and the like), ammonia, organic bases (e.g., trimethylamine, triethylamine, dicyclohexylamine, ethanolamine, diethanolamine, triethanolamine, meglumine, ethylenediamine, pyridine, picoline, quinoline, and the like), and amino acids, or salts of the compound represented by Formula (I) with inorganic acids (e.g., hydrochloric acid, sulfuric acid, nitric acid, carbonic acid, hydrobromic acid, phosphoric acid, hydroiodic acid, and the like), and organic acids (e.g., formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, trifluoroacetic acid, and the like). These salts can be formed by a conventional method.

The compound represented by Formula (I) contained in the pharmaceutical composition of the present invention or a pharmaceutically acceptable salt thereof may form a solvate (e.g., a hydrate), a cocrystal, and/or a crystal polymorph. The present invention also includes such various solvates, cocrystals, and crystal polymorphs. The "solvate" may be one wherein any number of solvent molecules (e.g., water molecules or the like) is coordinated with the compound represented by Formula (I). When the compound represented by Formula (I) or a pharmaceutically acceptable salt thereof is allowed to stand in the atmosphere, the compound may absorb water, resulting in attachment of adsorbed water or formation of a hydrate. Recrystallization of the compound represented by Formula (I) or a pharmaceutically acceptable salt thereof may form a crystal polymorph. The term "cocrystal" means that the compound represented by Formula (I) or a salt and a counter molecule are present in the same crystal lattice, and may contain any number of counter molecules.

The compound represented by Formula (I) contained in the pharmaceutical composition of the present invention or a pharmaceutically acceptable salt thereof may form a prodrug. The present invention also encompasses the pharmaceutical composition comprising any of such various prodrugs. The prodrug is a derivative of the compound of the present invention having a chemically or metabolically degradable group, and is a compound that becomes a pharmaceutically active compound of the present invention by solvolysis or under physiological conditions in vivo. The prodrug includes, for example, a compound that is converted to the compound represented by Formula (I) through enzymatic oxidation, reduction, hydrolysis, or the like under physiological conditions in vivo, and a compound that is converted to the compound represented by Formula (I) through hydrolysis by gastric acid or the like. Methods for selecting and producing an appropriate prodrug derivative are described in, for example, "Design of Prodrugs, Elsevier, Amsterdam, 1985". The prodrug may have activity in itself.

Since the compound of the present invention has an excellent ACC2-selective inhibitory effect, the compound of the present invention is also useful as a therapeutic and/or preventive agent for heart diseases, heart disease complications, skeletal muscle diseases, and skeletal muscle conditions, or as a therapeutic and/or preventive agent for triglyceride deposit cardiomyovasculopathy or skeletal muscle diseases or conditions caused by the accumulation of triglyceride in skeletal muscle cells.

Furthermore, the compound of the present invention has usefulness as a pharmaceutical, and preferably has any one or a plurality of the following excellent features.

a) Inhibitory effect against CYP enzymes (e.g., CYP1A2, CYP2C9, CYP2C19, CYP2D6, and CYP3A4) is weak.
b) Satisfactory pharmacokinetics such as high bioavailability and moderate clearance are exhibited.
c) Metabolic stability is high.
d) Irreversible inhibitory effect is not exhibited against CYP enzymes (e.g., CYP3A4) within the concentration range of the measurement conditions described in the present specification.
e) Mutagenicity is not exhibited.
f) The cardiovascular risk is low.
g) High solubility is exhibited.
h) Having no side effects of an increase in plasma triglyceride.
i) Insulin resistance is improved.
j) Having no side effects of a decrease in platelet concentration.

The pharmaceutical composition of the present invention can be administered orally or parenterally. Examples of a method of parenteral administration include dermal, subcutaneous, intravenous, intraarterial, intramuscular, intraperitoneal, transmucosal, inhalation, transnasal, ophthalmic, inner ear, or vaginal administration.

In the case of oral administration, the pharmaceutical composition may be prepared into any dosage form that is commonly used, such as a solid preparation for internal use (e.g., a tablet, a powder, a granule, a capsule, a pill, a film, or the like) or a liquid preparation for internal use (e.g., a suspension, an emulsion, an elixir, a syrup, a lemonade, a spirit, an aromatic water, an extract, a decoction, a tincture, or the like), and administered. The tablet may be a sugar-coated tablet, a film-coated tablet, an enteric-coated tablet, a sustained-release tablet, a troche tablet, a sublingual tablet, a buccal tablet, a chewable tablet, or an orally disintegrating tablet; the powder and the granule may be a dry syrup; and the capsule may be a soft capsule, a micro capsule, or a sustained-release capsule.

In the case of parenteral administration, the pharmaceutical composition can be suitably administered in any dosage form that is commonly used, such as an injection, an infusion, or a preparation for external use (e.g., an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder for external use, a suppository, or the like). The injection may be an emulsion of O/W, W/O, O/W/O, W/O/W type, or the like.

An effective amount of the compound of the present invention can be mixed, if necessary, with various pharmaceutical additives, such as an excipient, a binder, a disintegrant, and a lubricant, suitable for the dosage form to prepare a pharmaceutical composition. The pharmaceutical composition can be further prepared as a pharmaceutical composition for children, the elderly, patients with serious conditions, or operation by appropriately changing the effective amount of the compound of the present invention, the dosage form, and/or various pharmaceutical additives. For example, the pharmaceutical composition for children may be administered to patients who are neonates (younger than 4 weeks old after the birth), infants (4 weeks old after the birth to younger than 1 year old), infant children (1 year old or older and younger than 7 years old), children (7 years old or older and younger than 15 years old), or 15 to 18 years old. For example, the pharmaceutical composition for the elderly may be administered to patients who are 65 years old or older.

The dose of the pharmaceutical composition of the present invention is desirably set in consideration of the age or body weight of a patient, the type or severity of a disease, an administration route, and the like. In the case of oral administration, the dose is within a range of usually 0.05 to 100 mg/kg/day, preferably 0.1 to 10 mg/kg/day. Although varying depending on the administration route, the dose in the case of parenteral administration is within a range of

usually 0.005 to 10 mg/kg/day, preferably 0.01 to 1 mg/kg/day. The dosage may be administered once to several times a day.

A compound represented by Formula (I) contained in the pharmaceutical composition of the present invention:

[Chemical Formula 22]

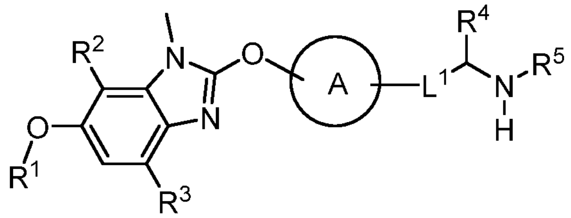

(I)

(each symbol has the same meaning as described above) or a pharmaceutically acceptable salt thereof can be used in combination with a diuretic, an ACE inhibitor, an angiotensin II receptor antagonist, an angiotensin receptor/neprilysin inhibitor, an aldosterone antagonist, a β-blocker, an SGLT2 inhibitor, and a vasodilator, for the purpose of, for example, enhancing the therapeutic and/or prophylactic action of the compound on heart diseases or heart disease complications or reducing the dose of the compound. For example, it can be used in combination with at least one compound selected from the group consisting of furosemide, enalapril, candesartan cilexetil, sacubitril/valsartan, spironolactone, carvedilol, dapagliflozin, and nitroglycerin, or a pharmaceutically acceptable salt thereof (hereinafter, referred to as concomitant drug 1). In this case, timing of administration of the compound of the present invention and the concomitant drug 1 is not limited, and these may be administered to a subject simultaneously or continuously, or at regular intervals. Furthermore, the compound of the present invention and the concomitant drug 1 may be administered as two or more preparations containing respective active ingredients, or may be administered as a single preparation containing these active ingredients.

A compound represented by Formula (I) contained in the pharmaceutical composition of the present invention:

[Chemical Formula 23]

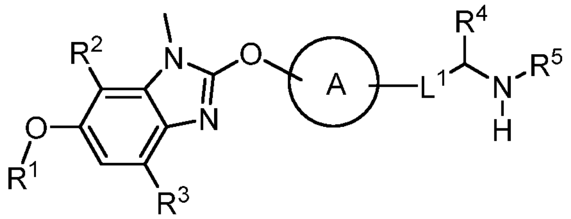

(I)

(each symbol has the same meaning as described above) or a pharmaceutically acceptable salt thereof can be used in combination with testosterone replacement therapy and/or amino acid supplementation (for example, branched-chain amino acid supplementation), myostatin neutralizing antibodies, or the like, enhancing the therapeutic and/or prophylactic action of the compound on skeletal muscle diseases or conditions (for example, liver cirrhosis with sarcopenia), or reducing the dose of the compound, or the like. Examples of the myostatin neutralizing antibody include domaglozumab, landogrozumab, stamulumab, and the like.

For example, the compound of the present invention can be used in combination with at least one compound selected from the group consisting of testosterone, branched chain amino acids, and myostatin neutralizing antibodies, or a pharmaceutically acceptable salt thereof (hereinafter, referred to as a concomitant drug 2). In this case, timing of administration of the compound of the present invention and the concomitant drug 2 is not limited, and these may be administered to a subject simultaneously or continuously, or at regular intervals. Furthermore, the compound of the present invention and the concomitant drug 2 may be administered as two or more preparations containing respective active ingredients, or may be administered as a single preparation containing these active ingredients.

For example, the compound of the present invention can be used in combination with at least one therapy selected from the group consisting of testosterone replacement therapy and amino acid supplementation (for example, branched-chain amino acid supplementation) (hereinafter referred to as concomitant therapy 1). In this case, the administration time of the compound of the present invention and the implementation time of the concomitant therapy 1 are not limited. The administration of the compound of the present invention to an administration subject may be performed

simultaneously or sequentially with the concomitant therapy 1, or at regular intervals.

The dose of the concomitant drug 1 or 2 can be appropriately selected on the basis of the clinically used dose. Furthermore, the mixing ratio of the compound of the present invention and the concomitant drug 1 or 2 can be appropriately selected in consideration of the subject of administration, administration route, target diseases, symptoms, combinations, and the like. For example, when the subject of administration is human, the concomitant drug may be used in a range of 0.01 to 100 parts by weight with respect to 1 part by weight of the compound of the present invention.

[EXAMPLES]

Hereinafter, the present invention will be described in more detail with reference to Examples and Test Examples, but the present invention is not limited thereto.

Preparation Example 1: Preparation of Recombinant Human ACC2

A cDNA encoding human ACC2 protein (27 amino acid residues to 2458 amino acid residues from the N-terminus) was cloned from a human kidney cDNA library (Clontech Laboratories, Inc.), a His-tag sequence was introduced at the 5' end, and then inserted into pFastBac1 (Invitrogen). A recombinant baculovirus was produced in accordance with the protocol for a Bac-to-Bac baculovirus expression system (Invitrogen), and Sf-9 cells were infected therewith to express human ACC2 protein. The recovered cells were crushed, filtered through a filter, and then subjected to Ni affinity chromatography and anion exchange chromatography. Fractions containing human ACC2 protein were collected to obtain recombinant human ACC2.

Preparation Example 2: Preparation of Recombinant Human ACC1

A cDNA encoding human ACC1 protein (1 amino acid residue to 2346 amino acid residues from the N-terminus) was cloned from a human liver cDNA library (BioChain Institute Inc.), myc tag and His-tag sequences were introduced at the 3' end, and then inserted into pIEXBAC3 (Novagen). A recombinant baculovirus was produced in accordance with the protocol for FlashBACGOLD (Oxford Expression Technologies Ltd.), and Sf-9 cells were infected therewith to express human ACC1 protein. The recovered cells were crushed, filtered through a filter, and then subjected to Ni affinity chromatography and anion exchange chromatography. Fractions containing human ACC1 protein were collected to obtain recombinant human ACC1.

Test Example 1: Measurement of Inhibitory Activity of Human ACC1 and ACC2

The recombinant human ACC1 and the recombinant human ACC2 obtained from the above Preparation Examples were preincubated in an assay buffer (50 mM HEPES-KOH (pH 7.4), 10 mM magnesium chloride, 6 to 10 mM potassium citrate, 4 mM glutathione reduced form, and 1.5 mg/ml bovine serum albumin) for 1 hour. Then, 5 $\mu$L of the pre-incubated enzyme solution and 5 $\mu$L of a substrate solution (50 mM HEPES-KOH (pH 7.4), 1 mM ATP, 0.8 mM acetyl-CoA, and 25 to 50 mM potassium bicarbonate) were added to a 384-well microplate into which 0.2 $\mu$L of each of solutions of the compound of the present invention (DMSO) had been dispensed, and the mixture was centrifuged and shaken, and then incubated in a wet box at room temperature for 1 to 3 hours. After incubation, the enzymatic reaction was stopped by addition of EDTA, and then the sample was co-crystallized with an $\alpha$-cyano-4-hydroxy cinnamic acid (CHCA) matrix on a MALDI target plate, and measurement was performed in a reflector negative mode using a matrix-assisted laser desorption ionization-time-of-flight mass spectrometer (MALDI-TOF MS). Deprotonated ions of the substrate acetyl-CoA (AcCoA) and the reaction product malonyl-CoA (MalCoA) were detected, and the intensity of each signal was used to calculate a conversion rate to malonyl-CoA or succinyl-CoA, i.e., intensity of [MalCoA-H]-/(intensity of [MalCoA-H]- + intensity of [AcCoA-H]-). A 50% inhibitory concentration (IC50 value) was calculated from the inhibition rate of the enzyme reaction at each compound concentration. The potassium citrate concentration in the assay buffer, the potassium bicarbonate concentration in the substrate solution, and the incubation time were adjusted within the above concentrations or reaction times for each lot of the enzyme to be used.

Results of Test Example 1 are shown below.

[Table 1]

| Compound No. | IC50 [$\mu$M] | |
|---|---|---|
| | hACC1 | hACC2 |
| I-1 | 0.32 | 0.0051 |

(continued)

| Compound No. | IC50 [$\mu$M] | |
|---|---|---|
| | hACC1 | hACC2 |
| I-2 | 2.4 | 0.01 |
| I-3 | 2.8 | 0.0066 |
| I-4 | 2.7 | 0.0047 |
| I-5 | 6.9 | 0.0089 |
| I-6 | 0.92 | 0.012 |
| I-7 | 25 | 0.18 |
| I-8 | 6.2 | 0.017 |
| I-9 | 2.6 | 0.013 |
| I-10 | >100 | 0.089 |
| I-11 | 7 | 0.0096 |
| I-12 | 6.9 | 0.0056 |
| I-13 | 0.91 | 0.0066 |
| I-14 | 2.7 | 0.0066 |
| I-15 | 1.7 | 0.0038 |

Test Example 2: Metabolic Stability Test

Commercially available pooled human liver microsomes were reacted with the compound of the present invention for a given time. A residual rate was calculated by comparison between the reacted sample and the unreacted sample to evaluate a degree at which the compound of the present invention is metabolized in the liver.

The compound was reacted at 37°C for 0 minutes or 30 minutes in the presence of 1 mmol/L NADPH in 0.2 mL of a buffer (50 mmol/L Tris-HCl pH 7.4, 150 mmol/L potassium chloride, and 10 mmol/L magnesium chloride) containing 0.5 mg protein/mL of the human liver microsomes (oxidative reaction). After the reaction, 50 $\mu$L of the reaction solution was added to 100 $\mu$L of a solution of methanol/acetonitrile = 1/1 (v/v) and mixed, and the mixture was centrifuged at 3000 rpm for 15 minutes. The compound of the present invention in the centrifuged supernatant was quantified by LC/MS/MS or solid-phase extraction (SPE)/MS. The amount of the compound of the present invention remained after the reaction was calculated with the amount of the compound at 0 minutes of the reaction defined as 100%.

Results of Test Example 2 are shown below.

[Table 2]

| Compound No. | Remaining amount of compound [%] | |
|---|---|---|
| | Human | Rat |
| I-3 | 80.3 | 82.6 |
| I-4 | 93.4 | 87.3 |
| I-5 | 98.9 | 83.4 |
| I-6 | 93.2 | 74 |
| I-7 | 89.1 | 74.7 |
| I-8 | 83.6 | 85.4 |
| I-11 | 95.8 | 94.5 |
| I-12 | 83.3 | 85.5 |
| I-13 | 95.4 | 87 |

(continued)

| Compound No. | Remaining amount of compound [%] | |
|---|---|---|
| | Human | Rat |
| I-14 | 94.8 | 78 |
| I-15 | 80.2 | 76.2 |

Adjustment Example 3: Generation of Atgl knockout mice

Atgl (adipose-triglyceride lipase) knockout mice are known as non-clinical models of TGCV. Atgl knockout mice (KO mice) deficient in the Atgl gene were each produced by a known method.

Test Example 3: Effect on long-chain fatty acid metabolizing ability in Atgl knockout mouse myocardium

The compound of the present invention was suspended in a 0.5% HPMC aqueous solution so that the dose of the same was 30 or 45 mg/kg/10 mL, and orally administered to 6 to 7-week-old male Atgl knockout mice on the day before and the day when the long-chain fatty acid metabolizing ability was measured. On the day of measurement of long-chain fatty acid metabolizing ability, 14.6 MBq/mouse of iodine-123-6 methyl iodophenyl-pen-tadecanoic acid (BMIPP) was administered via the tail vein. At 1 hour and 4 hours after administration, the mice were imaged by single-photon emission computed tomography (SPECT)/CT under anesthesia, and the radioactivity of the administered BMIPP in the myocardium was measured. The metabolism of long-chain fatty acids in the myocardium was calculated as a wash out rate by the following formula.

$$[(\text{BMIPP uptake (1 h) - remaining BMIPP (4 h))/BMIPP uptake (1 h)}]$$

(Results)

In this evaluation system, Compound 1-12 (30 mg/kg/10 mL) tended to improve the long-chain fatty acid metabolizing ability ($p = 0.096$, Welch's t test). In addition, Compound 1-13 (45 mg/kg/10 mL) showed a significant improving effect on the long-chain fatty acid metabolizing ability ($p < 0.05$, Welch's t test). Results of Test Example 3 are shown in Fig. 1.

Test Example 4: Evaluation of cardiac lipid content in Atgl knockout mice

In order to examine the influence of the compound of the present invention on cardiac lipid, hearts were collected after a vehicle or the compound of the present invention was administered to Atgl knockout mice. The collected heart was homogenized, lipid in the tissue was extracted using the Folch method described in Folch et al. (J Biol Chem. 1957 May; 226 (1): 497-509.) and the like, the amount of cardiac triglyceride (TG) and the amount of cardiac free fatty acid (NEFA) per tissue were calculated using Hitachi Automatic Analyzer 7180 (Hitachi High-Technologies Corporation), and the influence of the compound of the present invention on the cardiac lipid content was evaluated. As the reagents, Autocera (registered trademark) TG or Autocera (registered trademark) NEFA (Sekisui Medical Co., Ltd.) was used.

Test Example 5: Evaluation of cardiac function in Atgl knockout mice

In order to examine the influence of the compound of the present invention on cardiac function, left ventricular ejection fraction (LVEF) indicating the contractile force of the heart was evaluated using a high-resolution ultrasonic imaging system Vevo 770 (Primetech Corp.) after repeated administration of a vehicle or the compound of the present invention to Atgl knockout mice.

Test Example 6: Survival time evaluation regarding Atgl knockout mice

In order to examine the influence of the compound of the present invention on survival time, the influence on survival time was evaluated after a vehicle or the compound of the present invention was administered to Atgl knockout mice.

Test Example 7: Influence on Platelet Count

The compound of the present invention was suspended in a 0.5% methylcellulose solution so as to have a dose of 50 to 600 mg/kg/day, and was orally repeatedly administered for 4 days to 6-week-old male Crl:CD (SD) rats under non-fasted conditions. On day 5 from the start of administration, blood was collected from the abdominal vena cava under anesthesia, and plasma was collected. A platelet count in plasma was evaluated with an automatic hemocytometer.

Both Compound 1-12 and Compound 1-13 did not affect the platelet count in SD rats within a dose range used for evaluation. Results of Test Example 7 are shown below.

[Table 3]

| I-12 | Dose | 0 mg/kg/day | 100 mg/kg/day | 300 mg/kg/day | 600 mg/kg/day |
|---|---|---|---|---|---|
| | Platelet count (N=4) | 1055 ± 186 | 987 ± 86 | 988± 43 | 1061 ± 85 |
| Data: mean ± standard deviation | | | | | |

[Table 4]

| I-13 | **Dose** | 0 mg/kg/day | 50 mg/kg/day | 100 mg/kg/day | 200 mg/kg/day |
|---|---|---|---|---|---|
| | Platelet count (N=4) | 1164 ± 102 | 1079 ± 73 | 1048 ± 65 | 1122 ± 87 |
| Data: mean ± standard deviation | | | | | |

Test Example 8: Evaluation of plasma triglyceride concentration in mice fed ultra-high fat, choline-deficient, methionine-lowered diet

Six-week-old male C57BL/6JJcl mice were fed an ultra-high fat, choline-deficient, methionine-lowered diet (60 kcal% fat content, using lard, choline-deficient, methionine-lowered (0.1%)), and at the same time, the compound of the present invention was suspended in a vehicle (0.5% methylcellulose aqueous solution) so as to have a dose of 3 to 45 mg/kg/10 mL, and orally repeatedly administered (b.i.d.) for 8 weeks. Blood was collected from the tail vein during the repeated administration period, and biochemical parameters in plasma were measured.

(Results)

In both the Compound I-12-administered group and the Compound I-13-administered group, a plasma triglyceride concentration was lower than that in the vehicle-administered group. Results are shown in Figs. 2 and 3 and Tables 5 and 6.

[Table 5]

| Administration group | Plasma triglyceride concentration [mg/dL] | | |
|---|---|---|---|
| | 2 weeks | 4 weeks | 7 weeks |
| Vehicle | 30.1±1. 7 | 22.7±1.8 | 27.0±1.8 |
| I-12 (3 mg/kg, bid) | 32.9±1.3 | 22.4±2.1 | 27.2±1.5 |
| I-12 (10 mg/kg, bid) | 30.4±2.2 | 21.5±1.8 | 22.1±1.9 |
| I-12 (30 mg/kg, bid) | 23.1±2.5* | 11.1±1.1** | 16.2±1.6*** |
| Data: mean ± standard error<br>*: p < 0.05, **: p < 0.01, ***: p < 0.001 vs vehicle-administered group (Dunnett's test) | | | |

[Table 6]

| Administration group | Plasma triglyceride concentration [mg/dL] | | | | |
|---|---|---|---|---|---|
| | 0 weeks | 1 week | 3 weeks | 5 weeks | 7 weeks |
| Vehicle | 63.1±3.7 | 23.3±1.5 | 23.9±1.4 | 24.6±1.6 | 24.4±1.1 |
| I-13 (15 mg/kg, bid) | 61.6±3.1 | 23.7±1.0 | 19.8±1.2 | 19.0±1.7* | 18.8±1.2** |
| I-13 (45 mg/kg, bid) | 63.6±3.7 | 22.4±2.5 | 18.9±1.4* | 16.5±1.7** | 15.1±1.0*** |
| Data: mean ± standard error<br>*: p < 0.05, **: p < 0.01, ***: p < 0.001 vs vehicle-administered group (Dunnett's test) | | | | | |

Test Example 9: CYP Inhibition Test

Using commercially available pooled human liver microsomes, and employing, as indices, O-deethylation of 7-ethoxyresorufin (CYP1A2), methyl-hydroxylation of tolbutamide (CYP2C9), 4'-hydroxylation of mephenytoin (CYP2C19), O-demethylation of dextromethorphan (CYP2D6), and hydroxylation of terfenadine (CYP3A4), which are typical substrate metabolic reactions of five human major CYP5 molecular species (CYP1A2, 2C9, 2C19, 2D6, and 3A4), a degree at which an amount of each metabolite produced is inhibited by the compound of the present invention was assessed.

Reaction conditions are as follows: substrate, 0.5 μmol/L ethoxyresorufin (CYP1A2), 100 μmol/L tolbutamide (CYP2C9), 50 μmol/L S-mephenytoin (CYP2C19), 5 μmol/L dextromethorphan (CYP2D6), 1 μmol/L terfenadine (CYP3A4); reaction time, 15 minutes; reaction temperature, 37°C; enzyme, 0.2 mg protein/mL of pooled human liver microsomes; concentrations of the compound of the present invention, 1, 5, 10, 20 μmol/L (four points).

Each of the five substrates, the human liver microsomes, and the compound of the present invention were added according to the recipe described above into a 50 mmol/L Hepes buffer in a 96-well plate, and a coenzyme NADPH was added thereto to initiate the metabolic reactions serving as indices. After reaction at 37°C for 15 minutes, a solution of methanol/acetonitrile = 1/1 (V/V) was added to stop the reaction. After centrifugation at 3000 rpm for 15 minutes, resorufin (CYP1A2 metabolite) in the centrifuged supernatant was quantified with a fluorescence multilabel counter or by LC/MS/MS, and tolbutamide hydroxide (CYP2C9 metabolite), mephenytoin 4'-hydroxide (CYP2C19 metabolite), dextrorphan (CYP2D6 metabolite), and terfenadine alcohol (CYP3A4 metabolite) were quantified by LC/MS/MS.

Only DMSO as a solvent dissolving a compound instead of the compound of the present invention was added to the reaction solution, and the mixture was used as a control (100%). Remaining activity (%) was calculated, and $IC_{50}$ was calculated by inverse estimation based on a logistic model using the concentrations and the inhibition rates.

Test Example 10: CYP3A4 Fluorescent MBI Test

The CYP3A4 fluorescent MBI test is a test for examining the enhancement of CYP3A4 inhibition of the compound of the present invention by a metabolic reaction. 7-Benzyloxy trifluoromethyl coumarin (7-BFC) is debenzylated by a CYP3A4 enzyme (Escherichia coli-expressed enzyme) to produce a fluorescent metabolite, 7-hydroxy trifluoromethyl coumarin (7-HFC). CYP3A4 inhibition was evaluated using the 7-HFC production reaction as an index.

Reaction conditions are as follows: substrate, 5.6 μmol/L 7-BFC; pre-reaction time, 0 or 30 minutes; reaction time, 15 minutes; reaction temperature, 25°C (room temperature); CYP3A4 content (Escherichia coli-expressed enzyme), at pre-reaction 62.5 pmol/mL, at reaction 6.25 pmol/mL (at 10-fold dilution); concentrations of the compound of the present invention, 0.625, 1.25, 2.5, 5, 10, 20 μmol/L (six points).

An enzyme and a solution of the compound of the present invention were added according to the composition of the pre-reaction as described above into a K-Pi buffer (pH 7.4) as a pre-reaction solution in a 96-well plate. A part of the pre-reaction solution was transferred to another 96-well plate so as to be diluted by 1/10 with a substrate and a K-Pi buffer. NADPH as a coenzyme was added to initiate a reaction serving as an index (without pre-reaction). After reaction for a predetermined time, acetonitrile/0.5 mol/L Tris (trishydroxyaminomethane) = 4/1 (V/V) was added to stop the reaction. In addition, NADPH was added to the remaining pre-reaction solution to initiate a pre-reaction (with pre-reaction). After pre-reaction for a predetermined time, a part was transferred to another plate so as to be diluted by 1/10 with a substrate and a K-Pi buffer to initiate a reaction serving as an index. After reaction for a predetermined time, acetonitrile/0.5 mol/L Tris (trishydroxyaminomethane) = 4/1 (V/V) was added to stop the reaction. For the plate on which each index reaction had been performed, a fluorescent value of 7-HFC that is a metabolite was measured with a fluorescent plate reader. (Ex = 420 nm, Em = 535 nm). The dilution concentration and the dilution solvent were changed

as necessary.

Only DMSO as a solvent dissolving the compound of the present invention was added to the reaction system, and the mixture was used as a control (100%). Remaining activity (%) at the time of addition of the compound of the present invention at each concentration was calculated. $IC_{50}$ was calculated by inverse estimation based on a logistic model using the concentrations and the inhibition rates. A case where a difference in $IC_{50}$ values was 5 μmol/L or more was defined as (+). A case where the difference was 3 μmol/L or less was defined as (-).

Test Example 11: CYP3A4 (MDZ) MBI Test

This test evaluates, as to CYP3A4 inhibition of the compound of the present invention, mechanism based inhibition (MBI) ability from enhancement of an inhibitory effect caused by a metabolic reaction of the compound of the present invention. CYP3A4 inhibition was evaluated using pooled human liver microsomes with 1-hydroxylation reaction of midazolam (MDZ) as an index.

Reaction conditions are as follows: substrate, 10 μmol/L MDZ; pre-reaction time, 0 or 30 minutes; substrate metabolic reaction time, 2 minutes; reaction temperature, 37°C; pooled human liver microsomes, at pre-reaction 0.5 mg/mL, at reaction time 0.05 mg/mL (at 10-fold dilution); concentrations of the compound of the present invention at pre-reaction, 1, 5, 10, 20 μmol/L (four points).

The pooled human liver microsomes and a solution of the compound of the present invention were added according to the composition of the pre-reaction described above into a K-Pi buffer (pH 7.4) as a pre-reaction solution in a 96-well plate. A part of the pre-reaction solution was transferred to another 96-well plate so as to be diluted by 1/10 with a K-Pi buffer containing a substrate. NADPH as a coenzyme was added to initiate a reaction serving as an index (Preincubataion 0 min). After reaction for a predetermined time, a solution of methanol/acetonitrile = 1/1 (V/V) was added to stop the reaction. In addition, NADPH was added to the remaining pre-reaction solution to initiate a pre-reaction (Preincubataion 30 min). After pre-reaction for a predetermined time, a part was transferred to another plate so as to be diluted by 1/10 with a K-Pi buffer containing a substrate to initiate a reaction serving as an index. After reaction for a predetermined time, a solution of methanol/acetonitrile = 1/1 (V/V) was added to stop the reaction. The plate on which each index reaction had been performed was centrifuged at 3000 rpm for 15 minutes, and then 1-hydroxymidazolam in the centrifuged supernatant was quantified by LC/MS/MS. The dilution concentration and the dilution solvent were changed as necessary.

Only DMSO as a solvent dissolving a compound instead of the compound of the present invention was added to the reaction solution, and the mixture was used as a control (100%). Remaining activity (%) at the time of addition of the compound of the present invention at each concentration was calculated. IC was calculated by inverse estimation based on a logistic model using the concentrations and the inhibition rates. IC at Preincubataion 0 min/IC at Preincubataion 30 min was defined as a shifted IC value. A case where shifted IC was 1.5 or more was regarded as positive, and a case where shifted IC was 1.0 or less was regarded as negative.

Test Example 12: BA test

Experimental Material and Method to Study Oral Absorbability

(1) Animals used: Mice or rats were used.
(2) Breeding conditions: The mice or rats were allowed to freely take chow and sterilized tap water.
(3) Dose and grouping setting: A predetermined dose was orally administered and intravenously administered. Groups were set as follows: (dose was changed on a compound basis)

Oral administration: 2 to 60 μmol/kg or 1 to 30 mg/kg (n = 2 or 3)
Intravenous administration: 1 to 30 μmol/kg or 0.5 to 10 mg/kg (n = 2 or 3)

(4) Preparation of dosing solution: Oral administration was performed in a form of a solution or a suspension. Intravenous administration was performed after solubilization.
(5) Administration method: Oral administration was performed by gastric gavage using an oral sonde. Intravenous administration was performed via the tail vein through a syringe with an injection needle.
(6) Evaluation item: Blood was collected over time, and the concentration of the compound of the present invention in plasma was measured using LC/MS/MS.
(7) Statistical analysis: An area under plasma concentration-time curve (AUC) was calculated as to change in the concentration of the compound of the present invention in plasma by the moment analysis method, and bioavailability

(BA) of the compound of the present invention was calculated from a dose ratio and an AUC ratio between the oral administration group and the intravenous administration group.

The dilution concentration and the dilution solvent were changed as necessary.

Test Example 13: Clearance Evaluation Test

Experimental Material and Method

(1) Animals used: SD rats were used.
(2) Breeding conditions: The SD rats were allowed to freely take chow and sterilized tap water.
(3) Dose and grouping setting: A predetermined dose was intravenously administered. Groups were set as follows: Intravenous administration: 1 μmol/kg (n = 2)
(4) Preparation of dosing solution: Administration was performed after solubilization using a solvent of dimethyl sulfoxide/propylene glycol = 1/1.
(5) Administration method: Administration was performed via the tail vein through a syringe with an injection needle.
(6) Evaluation item: Blood was collected over time, and the concentration of the compound of the present invention in plasma was measured using LC/MS/MS.
(7) Statistical analysis: Total body clearance (CLtot) was calculated as to change in the concentration of the compound of the present invention in plasma by the moment analysis method. The dilution concentration and the dilution solvent were changed as necessary.

Test Example 14: Fluctuation Ames Test

Mutagenicity of the compound of the present invention is evaluated.

20 μL of cryopreserved Salmonella typhimurium (TA98 strain and TA100 strain) was inoculated to 10 mL of a liquid nutrient medium (2.5% Oxoid nutrient broth No. 2) and shake-precultured at 37°C for 10 hours. 7.70 to 8.00 mL of a bacterial solution of the TA98 strain was centrifuged (2000 × g, 10 minutes) to remove the culture solution. The bacterium was suspended in a Micro F buffer ($K_2HPO_4$: 3.5 g/L, $KH_2PO_4$: 1 g/L, $(NH_4)_2SO_4$: 1 g/L, trisodium citrate dihydrate: 0.25 g/L, and $MgSO_4 \cdot 7H_2O$: 0.1 g/L) in the same volume as that of the bacterial solution used in the centrifugation and added to 120 mL of an exposure medium (Micro F buffer containing biotin: 8 μg/mL, histidine: 0.2 μg/mL, and glucose: 8 mg/mL). 3.10 to 3.42 mL of a bacterial solution of the TA100 strain was added to 120 to 130 mL of an exposure medium to prepare a test bacterial solution. 12 μL each of a DMSO solution of the compound of the present invention (several serial dilutions from maximum dose 50 mg/mL at 2- to 3-fold common ratio), DMSO as a negative control, and 50 μg/mL of a 4-nitroquinoline-1-oxide DMSO solution for the TA98 strain and 0.25 μg/mL of a 2-(2-furyl)-3-(5-nitro-2-furyl)acrylamide DMSO solution for the TA100 strain under non-metabolic activation conditions or 40 μg/mL of a 2-aminoanthracene DMSO solution for the TA98 strain and 20 μg/mL of a 2-aminoanthracene DMSO solution for the TA100 strain under metabolic activation conditions as a positive control, and 588 μL of the test bacterial solution (a mixed solution of 498 μL of the test bacterial solution and 90 μL of S9 mix under metabolic activation conditions) were mixed, and the mixture was shake-cultured at 37°C for 90 minutes. 460 μL of the bacterial solution exposed to the compound of the present invention was mixed with 2300 μL of an indicator medium (Micro F buffer containing biotin: 8 μg/mL, histidine: 0.2 μg/mL, glucose: 8 mg/mL, and bromocresol purple: 37.5 μg/mL), and the mixture was dispensed into 48 wells/dose of a microplate at 50 pL/well and statically cultured at 37°C for 3 days. Since a well containing a bacterium that has acquired proliferation ability by mutation of an amino acid (histidine) synthase gene turns from purple to yellow due to a pH change, the bacterium proliferation well which had turned to yellow in 48 wells per dose was counted, and was evaluated by comparison with the negative control group. Negative mutagenicity was indicated as (-), and positive mutagenicity was indicated as (+). The dilution concentration and the dilution solvent were changed as necessary.

Test Example 15: hERG Test

For the purpose of evaluating a risk of an electrocardiogram QT interval prolongation of the compound of the present invention, an effect of the compound of the present invention on delayed rectifier $K^+$ current ($I_{Kr}$), which plays an important role in a ventricular repolarization process, was studied using CHO cells expressing human ether-a-go-go related gene (hERG) channel.

The cells were kept at a membrane potential of -80 mV by a whole cell patch clamp method using a fully

automated patch clamp system (QPatch; Sophion Bioscience A/S), given a leak potential of -50 mV, and then given depolarization stimulation of +20 mV for 2 seconds and further repolarization stimulation of -50 mV for 2 seconds. $I_{Kr}$ induced by this procedure was recorded. An extracellular fluid (NaCl: 145 mmol/L, KCl: 4 mmol/L, $CaCl_2$: 2 mmol/L, $MgCl_2$: 1 mmol/L, glucose: 10 mmol/L, HEPES (4-(2-hydroxyethyl)-1-piperazineethanesulfonic acid): 10 mmol/L, pH = 7.4) with dimethyl sulfoxide concentration adjusted to 0.1% was used as a vehicle to apply the vehicle and the extracellular fluid containing the compound of the present invention dissolved at a concentration of interest to the cells under conditions of room temperature for 7 minutes or longer. From the obtained $I_{Kr}$, an absolute value of a maximum tail current based on a current value at the membrane potential where the cells had been kept was measured using analytical software (QPatch Assay software; Sophion Bioscience A/S). Furthermore, a maximum tail current after the application of the compound of the present invention with respect to a maximum tail current after the application of the vehicle was calculated as an inhibition rate to evaluate an influence of the compound of the present invention on $I_{Kr}$. The dilution concentration and the dilution solvent were changed as necessary.

Test Example 16: Ames Test

Mutagenicity of the compound of the present invention was evaluated by an Ames test with Salmonella typhimurium TA98, TA100, TA1535, and TA1537 strains and an Escherichia coli WP2uvrA strain as test bacterial strains. 0.1 mL of a DMSO solution of the compound of the present invention was mixed with 0.5 mL of S9 mix under metabolic activation conditions or 0.5 mL of a phosphate buffer under non-metabolic activation conditions and 0.1 mL of the test bacterial solution, and the mixture was overlaid on a minimum glucose agar plate together with 2 mL of soft agar for overlay containing histidine and biotin, or tryptophan. At the same time, the same procedure was performed for a negative control substance (DMSO) and a positive control substance (2-(2-furyl)-3-(5-nitro-2-furyl)acrylamide, sodium azide, 9-aminoacridine, or 2-aminoanthracene). After culture at 37°C for 48 hours, revertant colonies that had appeared were counted and evaluated by comparison with the negative control group. When the number of revertant colonies increased in a concentration-dependent manner and became twice or more the number of colonies of the negative control group, mutagenicity was determined to be positive (+). The dilution concentration and the dilution solvent were changed as necessary.

Test Example 17: Photohemolysis Test

The compound of the present invention is dissolved at a concentration of interest. The solution is mixed with 0.1 to 0.0008% concentrations of a red blood cell suspension (2.5 v/v%) prepared from sheep defibrinated blood on a microplate. The mixtures are irradiated with light (10 $J/cm^2$, 290 to 400 nm) in UVA and UVB regions using an ultraviolet fluorescent lamp (GL20SE lamp, Sankyo Electronics Co., Ltd. and FL20S-BLB lamp, Panasonic Corporation). The mixed solutions after completion of light irradiation are collected and centrifuged. The supernatant after the centrifugation is collected and transferred to a microplate. Then, absorbance (540 or 630 nm) of the supernatant is measured, and determination based on the absorbance is performed. The absorbance at 540 and 630 nm is used as an index for biomembrane injury (rate of photohemolysis (%)) and lipid membrane peroxidation (methemoglobin production), respectively. A case where the rate of photohemolysis is less than 10% and an amount of change in absorbance at 630 nm is less than 0.05 is defined as (-). A case where the rate of photohemolysis is 10% or more or an amount of change in absorbance at 630 nm is 0.05 or more is defined as (+).

Test Example 18: P-gp Substrate Test

The compound of the present invention was added to one side of Transwell (registered trademark, Corning Incorporated) where human MDR1-expressing cells or parent cells had been monolayer-cultured. The cells were reacted for a given time. Membrane permeability coefficients from the apical side toward the basolateral side (A $\rightarrow$ B) and from the basolateral side toward the apical side (B $\rightarrow$ A) are calculated for the MDR1-expressing cells or the parent cells, and efflux ratio (ER; ratio of the membrane permeability coefficient of B $\rightarrow$ A to that of A $\rightarrow$ B) values of the MDR1-expressing cells and the parent cells were calculated. The efflux ratios (ER values) of MDR1-expressing cells and the parent cells were compared to determine whether the compound of the present invention was a P-gp substrate.

Test Example 19: Solubility Test

Solubility of the compound of the present invention was determined under conditions of 1% DMSO addition. A 10 mmol/L compound solution was prepared with DMSO. 2 $\mu$L of the solution of the compound of the present invention was added to 198 $\mu$L each of a JP-1 solution and a JP-2 solution. After shaking at room temperature for 1 hour, the mixed solutions were filtered by suction. The filtrates were diluted 10- or 100-fold with methanol/water = 1/1 (V/V) or

acetonitrile/methanol/water = 1/1/2 (V/V/V), and concentrations in the filtrates were measured by an absolute calibration curve method using LC/MS or solid-phase extraction (SPE)/MS. The dilution concentration and the dilution solvent were changed as necessary.

The composition of the JP-1 solution is as follows.

Water was added to 2.0 g of sodium chloride and 7.0 mL of hydrochloric acid to make 1000 mL.
The composition of the JP-2 solution is as follows.
3.40 g of potassium dihydrogen phosphate and 3.55 g of dibasic sodium phosphate anhydrous were dissolved in water to make 1000 mL, and to 1 volume of the resultant, 1 volume of water was added.

Test Example 20: Powder Solubility Test

An appropriate amount of the compound of the present invention was placed in appropriate containers, and 200 μL each of a JP-1 solution (water was added to 2.0 g of sodium chloride and 7.0 mL of hydrochloric acid to make 1000 mL), a JP-2 solution (3.40 g of potassium dihydrogen phosphate and 3.55 g of dibasic sodium phosphate anhydrous were dissolved in water to make 1000 mL, and to 1 volume of the resultant, 1 volume of water was added), and 20 mmol/L sodium taurocholate (TCA)/JP-2 solution (a JP-2 solution was added to 1.08 g of TCA to make 100 mL) was added to each container. When the whole amount was dissolved after addition of the test solution, the compound of the present invention was appropriately added. The containers were hermetically sealed, shaken at 37°C for 1 hour, and then filtered. Each filtrate was diluted 2-fold by addition of 100 μL of methanol to 100 μL of the filtrate. The dilution rate was changed as necessary. The absence of air bubbles and precipitates was confirmed, and the containers were hermetically sealed and shaken. The compound of the present invention was quantified by an absolute calibration curve method using HPLC. The dilution concentration and the dilution solvent were changed as necessary.

Test Example 21: Visual Solubility Test

About 5 mg of the compound is weighed into three trace tubes, and each vehicle (water for injection, saline injection, 0.5% glucose solution) is added so as to have a compound concentration of 20%. After stirring by vortexing, the presence or absence of dissolution is visually confirmed. If the compound is dissolved, solubility in the vehicle is determined to be >20%. Each vehicle (water for injection, saline injection, glucose solution) is further added to these test solutions to prepare test solutions having a compound concentration of 10%, and after stirring by vortexing, the presence or absence of dissolution is visually confirmed. If the compound is dissolved, solubility in the vehicle is determined to be 20% to 10%. Similarly, the test is performed for up to a concentration of 5%, a concentration of 2.5%, and a concentration of 1%, and when the compound is not dissolved at a concentration of 1%, solubility in the vehicle is determined to be <1%. A pH at a concentration of 1% of the test solution is measured and recorded. The dilution concentration and the dilution solvent may be changed as necessary.

Test Example 22: pKa Measurement (Method for Measuring Capillary Electrophoresis Method (CE Method))

This is a method using a capillary zone electrophoresis technique and a separation method utilizing free electrophoresis of each sample component in a buffer containing an electrolyte.
When a compound solution is injected into a fused silica capillary filled with a buffer adjusted to pH 2.5 to 11.5 and then a high voltage (inlet side +, outlet side -) is applied to the capillary, the compound migrates at a rate reflecting the ionization state at the pH of the buffer (fast for the positively (+) charged compound, slowly for the negatively (-) charged compound). A difference between a migration time of this compound and a migration time of a neutral molecule (DMSO) was plotted against pH, and pKa was calculated after fitting. Measurement conditions are shown below.

Apparatus used: Beckman P/ACE system MDQ PDA
Electrophoretic liquid: buffer at pH 2.5 to 11.5 (containing 10 vol% MeOH)
Sample solution: (Blank) mixed solution of 10 μL of DMSO + 90 μL of water for injection
(Sample) 4 μL of 10 mM DMSO stock solution + 6 μL of DMSO + 90 μL of water for injection

(Method)

Capillary: fused silica capillary (Beckman Coulter, Inc., inner diameter of 50 μm, total length of 30.2 cm, effective

length of 20.0 cm)
Applied voltage: 10 kV (331 V/cm)
Applied air pressure: 0.7 psi
Capillary temperature: 25°C
Electroosmotic flow marker: DMSO
Detection: ultraviolet multi-wavelength absorption detection (measurement wavelength; 215 nm, 238 nm)
Sample injection: pressurization method (0.5 psi, 5 seconds)

Test Example 23: Evaluation of lipid in skeletal muscle cells (intramyocellular lipid (IMCL)) in SD rats

In order to examine the influence of Compound 1-13 on lipid in skeletal muscle cells (IMCL), 24 hours after a single oral administration of a vehicle or Compound I-13 (20 mg/kg) to SD rats, MR spectroscopy (MRS) data of the tibialis anterior muscle was measured using animal MRI (Varian MRI System 7T/210, Agilent Technologies) under isoflurane anesthesia. The measurement data was analyzed using software for quantitative analysis of MRS data (LCModel), and the quantity of IMCL was calculated as a creatine ratio.

(Results)

Compound 1-13 (20 mg/kg) showed a significant IMCL decreasing effect in SD rats. Results of Test Example 23 are shown in Fig. 4.

Test Example 24: Evaluation of lipid in skeletal muscle cells (IMCL) in diabetes model mice

In order to examine the influence of Compound 1-12 on lipid in skeletal muscle cells (IMCL) of diabetes model mice, 6 hours after a single oral administration of a vehicle or Compound 1-12 (15 mg/kg) to leptin receptor-deficient mice (Leprdb/Leprdb, db/db; db/db mice), MR spectroscopy (MRS) data of the tibialis anterior muscle was measured using animal MRI (Varian MRI System 7T/210, Agilent Technologies) under isoflurane anesthesia. The measurement data was analyzed using software for quantitative analysis of MRS data (LCModel), and the quantity of IMCL was calculated as a creatine ratio.

(Results)

Compound 1-12 (15 mg/kg) showed a significant IMCL decreasing effect in db/db mice. Results of Test Example 24 are shown in Fig. 5.

Test Example 25: Evaluation of lipid in skeletal muscle cells (IMCL) in diabetes model rats

In order to examine the influence of Compound 1-12 on lipid in skeletal muscle cells (IMCL) of diabetic model rats, vehicle or Compound 1-12 was orally administered to a spontaneous diabetes model (ZDF rats) twice a day (first morning administration: 7 mg/kg; second afternoon administration: 10 or 23 mg/kg), and 24 hours after the first administration, MR spectroscopy (MRS) data of the tibialis anterior muscle were measured using animal MRI (Varian MRI System 7T/210, Agilent Technologies) under isoflurane anesthesia. The measurement data was analyzed using software for quantitative analysis of MRS data (LCModel), and the quantity of IMCL was calculated as a creatine ratio.

(Results)

Compound 1-12 showed a significant IMCL decreasing effect in ZDF rats. Results of Test Example 25 are shown in Fig. 6.
Test Example 26: Evaluation of lipid in skeletal muscle cells (IMCL) and lipid outside skeletal muscle cells (extramyocellular lipid (EMCL)) in high-fat-diet-challenged mice
In order to examine the influence of Compound 1-14 on lipid in skeletal muscle cells (IMCL) and lipid outside skeletal muscle cells (EMCL) of high-fat-diet-challenged mice, vehicle or Compound 1-14 (5 mg/kg) was orally administered once a day repeatedly for 5 weeks to C57BL/6 mice (DIO mice) challenged with a high-fat diet for 27 weeks, and then, MR spectroscopy (MRS) data of the tibialis anterior muscle was measured using animal MRI (Varian MRI System 7T/210, Agilent Technologies) under isoflurane anesthesia. The measurement data was analyzed using software for quantitative analysis of MRS data (LCModel), and the quantities of IMCL and EMCL were calculated as a creatine ratio.

(Results)

Compound 1-14 showed a significant IMCL decreasing effect in DIO mice. On the other hand, EMCL was not significantly affected. Results of Test Example 26 are shown in Fig. 7.

Test Example 27: Evaluation of lipid in skeletal muscle cells (IMCL) and lipid outside skeletal muscle cells (extramyocellular lipid (EMCL)) in ACC2 knockout mice

In order to examine the influence of ACC2 deficiency on lipid in skeletal muscle cells (IMCL) and lipid outside skeletal muscle cells (EMCL), MR spectroscopy (MRS) data of the tibialis anterior muscle of wild-type mice and ACC2 knockout mice were measured using animal MRI (Varian MRI System 7T/210, Agilent Technologies) under isoflurane anesthesia, before high-fat diet loading, after high-fat diet loading for 3 days, and after high-fat diet loading for 12 weeks. The measurement data was analyzed using software for quantitative analysis of MRS data (LCModel), and the quantities of IMCL and EMCL were calculated as a creatine ratio.

(Results)

ACC2 knockout mice showed a significant decrease in IMCL before and after high-fat-diet challenging as compared to wild-type mice. On the other hand, regarding EMCL, no significant difference was observed between the wild-type mice and the ACC2 knockout mice. Results of Test Example 27 are shown in Fig. 8.

Test Example 28: Evaluation of spontaneous motility regarding Atgl knockout mice

In order to examine the influence of the compounds of the present invention on spontaneous motility, spontaneous motility of Atgl knockout mice was measured using SCANET MV-40 (MELQUEST) at 12 weeks of age after repeated administration of vehicle or the compounds of the present invention to the Atgl knockout mice. The mice were allowed to stand in a cage placed in the device, and the motility thereof for 30 minutes was measured.

(Results)

Compound 1-13 (45 mg/kg/10 mL) showed a significant improving effect on the reduction in the spontaneous motility observed in Atgl knockout mice ($p < 0.05$, Welch's t test). Results of Test Example 28 are shown in Fig. 9.

Test Example 29: Evaluation of spontaneous motility of Atgl knockout mice and Atgl-ACC2 double knockout mice

To investigate the effect of ACC2 deficiency on spontaneous motility, spontaneous motility was measured in 12-week-old wild-type mice, Atgl knockout mice, and Atgl-ACC2 double knockout mice, using SCANET MV-40 (MELQUEST). The mice were allowed to stand in a cage placed in the device, and the motility thereof for 30 minutes was measured.

(Results)

The defect of ACC showed a significant improving effect on the reduction in the spontaneous motility observed in Atgl knockout mice ($p < 0.05$, Welch's t test). Results of Test Example 29 are shown in Fig. 10.

The following Formulation Examples are merely examples and not intended to limit the scope of the invention.

The pharmaceutical composition of the present invention can be administered by any conventional route, particularly enterally, for example, orally, for example, in a form of a tablet or a capsule; parenterally, for example, in a form of an injectable preparation or a suspension; and topically, for example, in a form of a lotion, a gel, an ointment, or a cream, or in a transnasal form or a suppository form. The pharmaceutical composition of the present invention containing the compound in a free form or in a form of a pharmaceutically acceptable salt can be produced together with at least one pharmaceutically acceptable carrier or diluent in a conventional manner by a mixing, granulating, or coating method. For example, the oral composition can be a tablet, a granule, or a capsule, each containing an excipient, a disintegrant, a binder, a lubricant, and the like, as well as an active ingredient and the like. Furthermore, the injectable composition can be a solution or a suspension, may be sterilized, and may contain a preservative, a stabilizer, a buffering agent, and the like.

[INDUSTRIAL APPLICABILITY]

The method of the present invention for treating and/or preventing at least one of heart diseases, heart disease complications, skeletal muscle diseases, and skeletal muscle conditions, and the pharmaceutical composition for treatment used therefor are considered to exhibit an excellent therapeutic effect by administering a predetermined amount of an active ingredient, the compound represented by Formula (I) or a pharmaceutically acceptable salt thereof, to patients. In addition, having no side effects such as an increase in plasma triglyceride and a decrease in platelet concentration by administration of the compound represented by Formula (I) or a pharmaceutically acceptable salt thereof, the method of treatment and/or prevention and the pharmaceutical composition for treatment of the present invention can be applied extremely safely, and are also suitable for long-term administration. Therefore, they are extremely excellent method of treatment and/or prevention and pharmaceutical composition for treatment.

## Claims

1. A pharmaceutical composition for treating and/or preventing at least one of heart diseases and heart disease complications, the pharmaceutical composition comprising a compound represented by Formula (I):

[Chemical Formula 1]

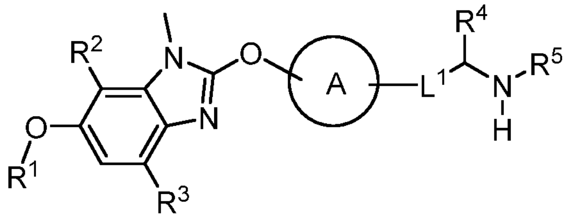

(I)

wherein $R^1$ is haloalkyl or non-aromatic carbocyclyl,
$R^2$ is a hydrogen atom or halogen,
$R^3$ is halogen,
ring A is a group represented by the formula:

[Chemical Formula 2],

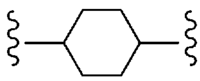 , 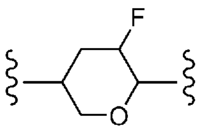 , 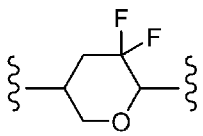 , 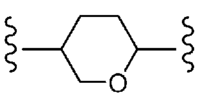 or 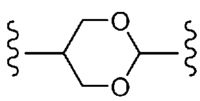

$-L^1-$ is $-O-(CH_2)-$, $-(CH_2)_2-$, $-(CH_2)-(CF_2)-$, or $-(CF_2)-(CH_2)-$ (wherein a left bond is attached to the ring A and a right bond is attached to a group represented by the formula:

[Chemical Formula 3]

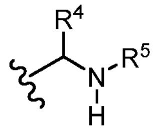

),

$R^4$ is alkyl or haloalkyl, and
$R^5$ is alkylcarbonyl or carbamoyl,
or a pharmaceutically acceptable salt thereof.

2. The pharmaceutical composition according to claim 1, wherein $R^1$ is non-aromatic carbocyclyl.

3. The pharmaceutical composition according to claim 1 or 2, wherein $R^2$ is a hydrogen atom.

4. The pharmaceutical composition according to any one of claims 1 to 3, wherein the ring A is a group represented by the formula:

[Chemical Formula 4]

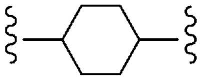

or

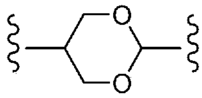

.

**5.** The pharmaceutical composition according to any one of claims 1 to 4, wherein $-L^1-$ is $-O-(CH_2)-$ or $-(CH_2)_2-$.

**6.** The pharmaceutical composition according to any one of claims 1 to 5, wherein $R^4$ is alkyl.

**7.** The pharmaceutical composition according to any one of claims 1 to 6, wherein $R^5$ is methylcarbonyl or carbamoyl.

**8.** The pharmaceutical composition according to claim 1, wherein the compound represented by Formula (I) or the pharmaceutically acceptable salt thereof is a compound selected from the group consisting of the following formulae:

[Chemical Formula 5]

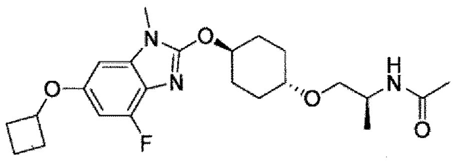
,
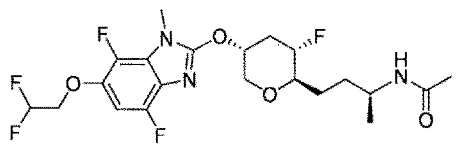
,
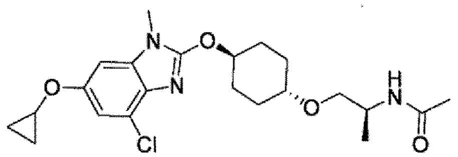
,
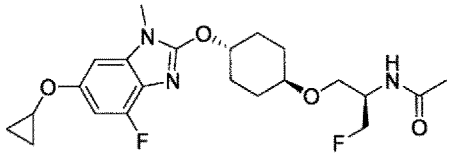
,
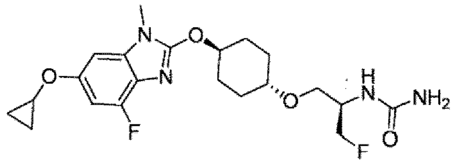
,
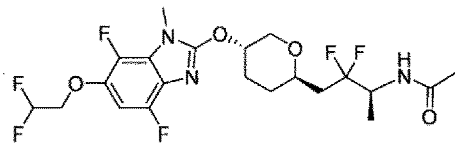
,
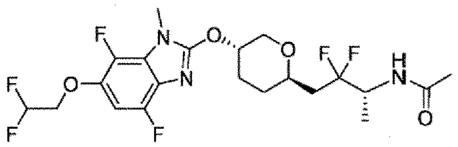
,
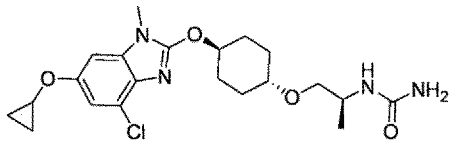
,
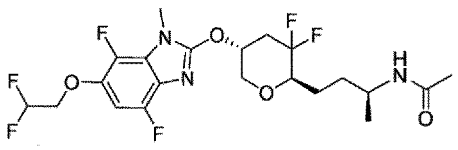
,
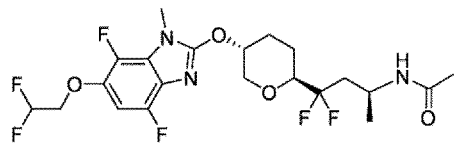
,
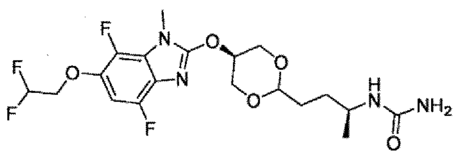
,
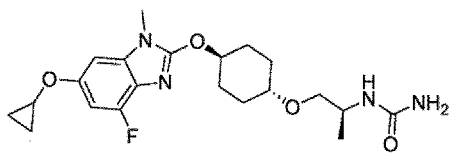
,
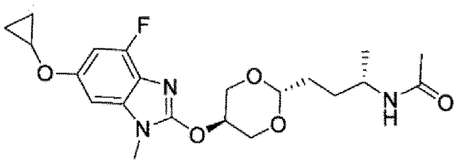
,
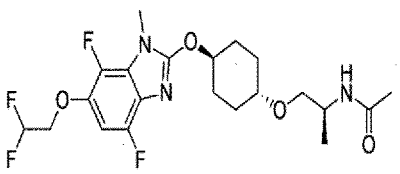

and

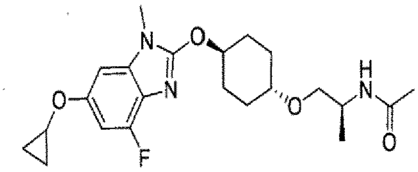

or a pharmaceutically acceptable salt thereof.

**9.** The pharmaceutical composition according to any one of claims 1 to 8, wherein the heart disease and heart disease complication are at least one disease selected from the group consisting of heart failure, angina pectoris, myocardial infarction, cardiomyopathy, arrhythmia, coronary artery disease, and skeletal muscle myopathy.

**10.** The pharmaceutical composition containing the compound according to any one of claims 1 to 8 for treating and/or preventing a disease caused by accumulation of triglyceride in at least one of cardiomyocytes, vascular smooth muscle cells, endothelial cells, skeletal muscle cells, polymorphonuclear leukocytes, renal tubular epithelial cells,

and pancreatic islet cells.

**11.** The pharmaceutical composition according to claim 10, wherein the disease is at least one disease selected from the group consisting of heart failure, angina pectoris, myocardial infarction, cardiomyopathy, arrhythmia, coronary artery disease, skeletal muscle myopathy, chronic kidney disease, and impaired glucose tolerance.

**12.** The pharmaceutical composition according to any one of claims 1 to 11, administration of which causes no side effects of an increase in plasma triglyceride.

**13.** The pharmaceutical composition according to any one of claims 1 to 11, administration of which causes no side effects of a decrease in platelet concentration.

**14.** A pharmaceutical composition for treating and/or preventing a skeletal muscle disease or condition, the pharmaceutical composition comprising a compound represented by Formula (I):

[Chemical Formula 6]

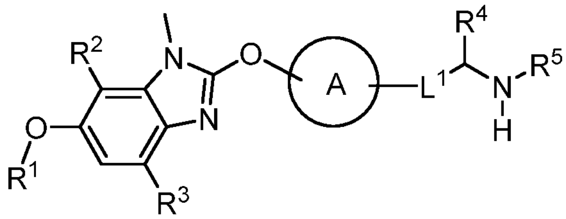

(I)

wherein $R^1$ is haloalkyl or non-aromatic carbocyclyl,
$R^2$ is a hydrogen atom or halogen,
$R^3$ is halogen,
ring A is a group represented by the formula:

[Chemical Formula 7],

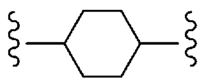 , 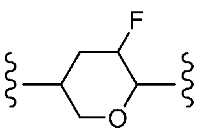 , 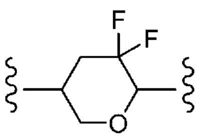 , 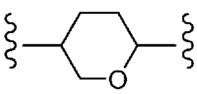 or 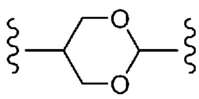

$-L^1-$ is $-O-(CH_2)-$, $-(CH_2)_2-$, $-(CH_2)-(CF_2)-$, or $-(CF_2)-(CH_2)-$ (wherein a left bond is attached to the ring A and a right bond is attached to a group represented by the formula:

[Chemical Formula 8]

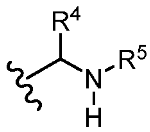 ),

$R^4$ is alkyl or haloalkyl, and
$R^5$ is alkylcarbonyl or carbamoyl,
or a pharmaceutically acceptable salt thereof.

**15.** The pharmaceutical composition according to claim 14, wherein $R^1$ is non-aromatic carbocyclyl.

**16.** The pharmaceutical composition according to claim 14 or 15, wherein $R^2$ is a hydrogen atom.

**17.** The pharmaceutical composition according to any one of claims 14 to 16, wherein the ring A is a group represented by the formula:

[Chemical Formula 9]

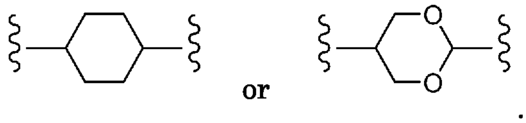

**18.** The pharmaceutical composition according to any one of claims 14 to 17, wherein $-L^1-$ is $-O-(CH_2)-$ or $-(CH_2)_2-$.

**19.** The pharmaceutical composition according to any one of claims 14 to 18, wherein $R^4$ is alkyl.

**20.** The pharmaceutical composition according to any one of claims 14 to 19, wherein $R^5$ is methylcarbonyl or carbamoyl.

**21.** The pharmaceutical composition according to claim 14, wherein the compound represented by Formula (I) or the pharmaceutically acceptable salt thereof is a compound selected from the group consisting of the following formulae:

[Chemical Formula 10]

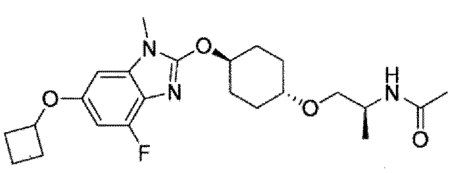 , 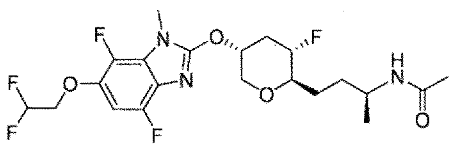 ,

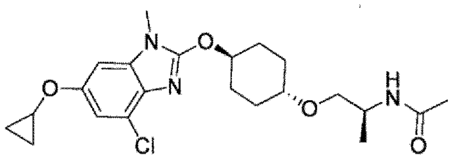 , 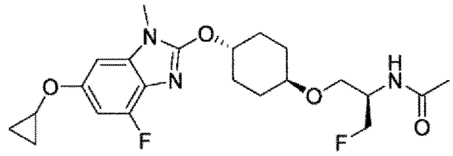 ,

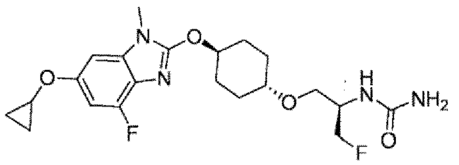 , 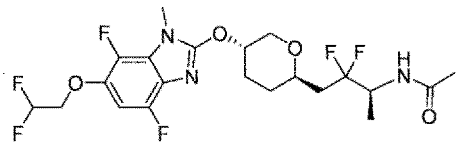 ,

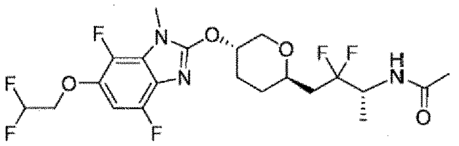 , 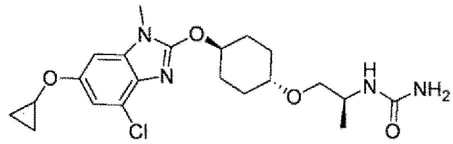 ,

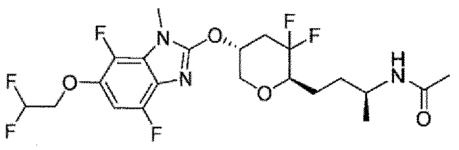 , 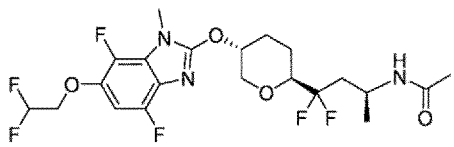 ,

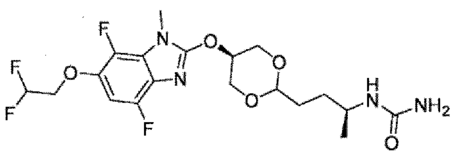 , 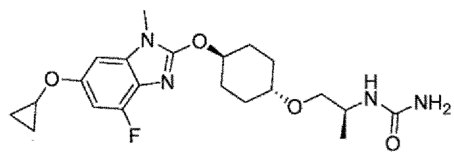 ,

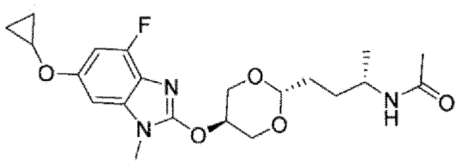 , 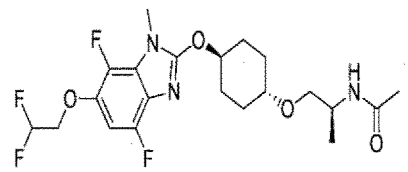

and 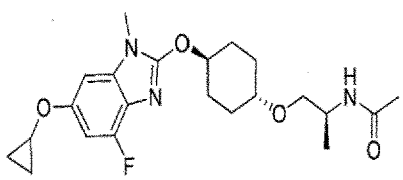

or a pharmaceutically acceptable salt thereof.

**22.** The pharmaceutical composition according to any one of claims 14 to 21, wherein the skeletal muscle disease or condition is caused by accumulation of triglyceride in skeletal muscle cells.

**23.** The pharmaceutical composition according to any one of claims 14 to 22, wherein the skeletal muscle disease or condition is skeletal muscle myopathy or sarcopenia.

**24.** The pharmaceutical composition according to any one of claims 14 to 23, wherein a pathology and/or symptom of the skeletal muscle disease or condition is due to a contribution of ACC2 activity.

**25.** The pharmaceutical composition according to claim 24, wherein the ACC2 activity contributes to a condition selected from the group consisting of diabetes, obesity and liver cirrhosis.

**26.** The pharmaceutical composition according to any one of claims 14 to 25, wherein the skeletal muscle disease or condition is associated with a condition selected from the group consisting of diabetes, obesity, and liver cirrhosis.

**27.** The pharmaceutical composition according to claim 26, wherein the skeletal muscle disease or condition is associated with liver cirrhosis.

**28.** The pharmaceutical composition according to any one of claims 14 to 27, wherein the pharmaceutical composition maintains or increases skeletal muscle function in a subject having the skeletal muscle disease or condition.

**29.** The pharmaceutical composition according to any one of claims 14 to 27, wherein the pharmaceutical composition promotes skeletal muscle recovery in a subject having the skeletal muscle disease or condition.

[Fig. 1]

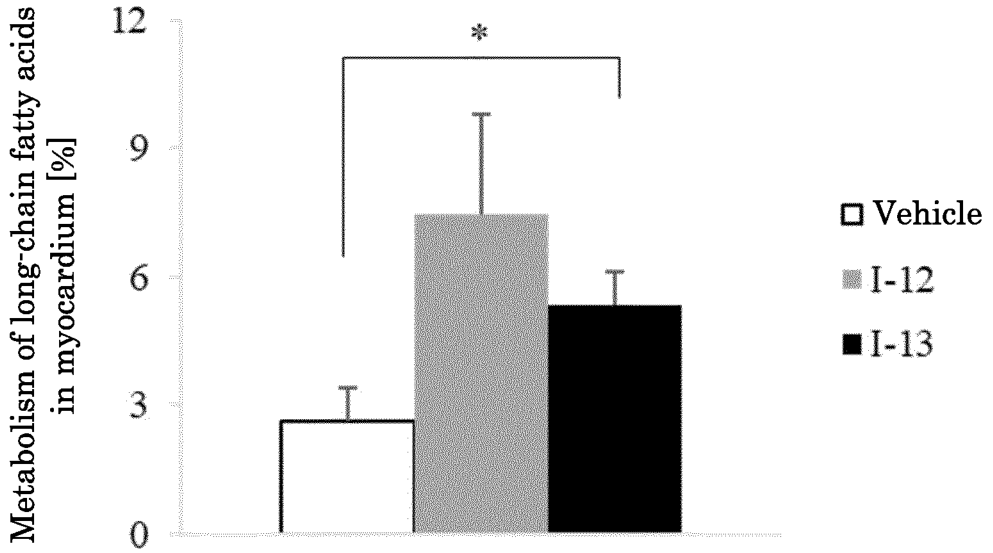
Metabolism of long-chain fatty acids in myocardium [%]
12
9
6
3
0
*
Vehicle
I-12
I-13

[Fig. 2]

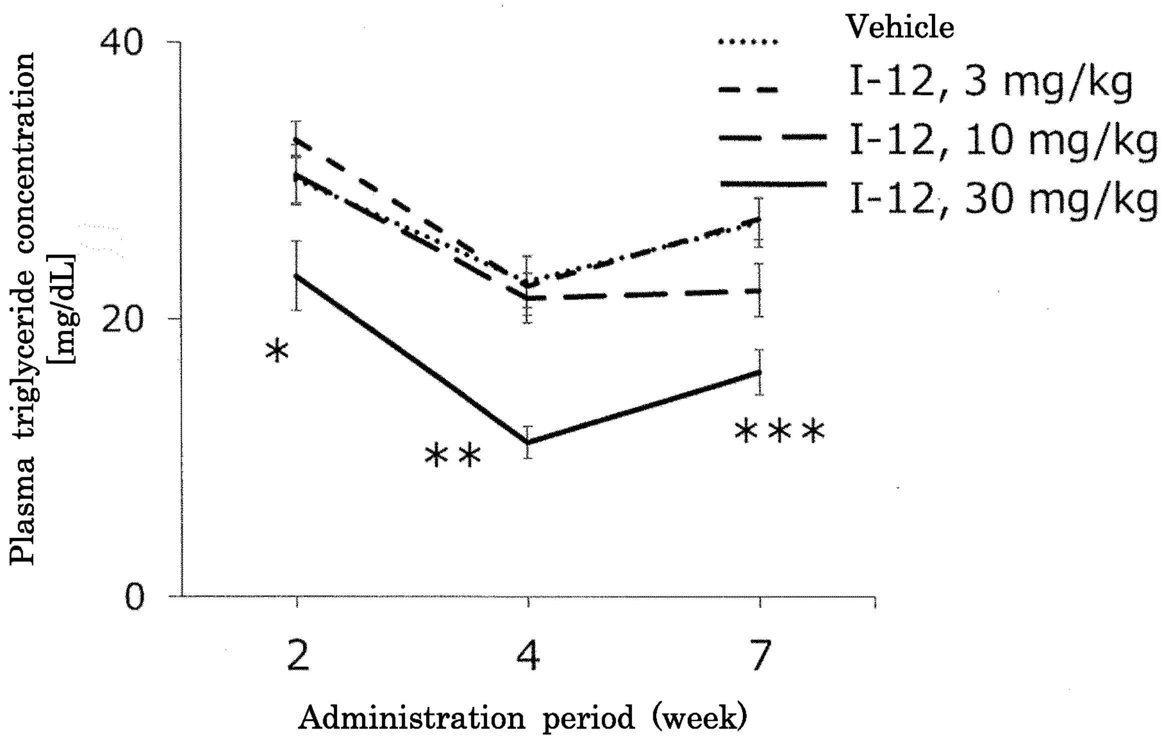
Plasma triglyceride concentration [mg/dL]
40
20
0
Vehicle
I-12, 3 mg/kg
I-12, 10 mg/kg
I-12, 30 mg/kg
*
**
***
2
4
7
Administration period (week)

[Fig. 3]

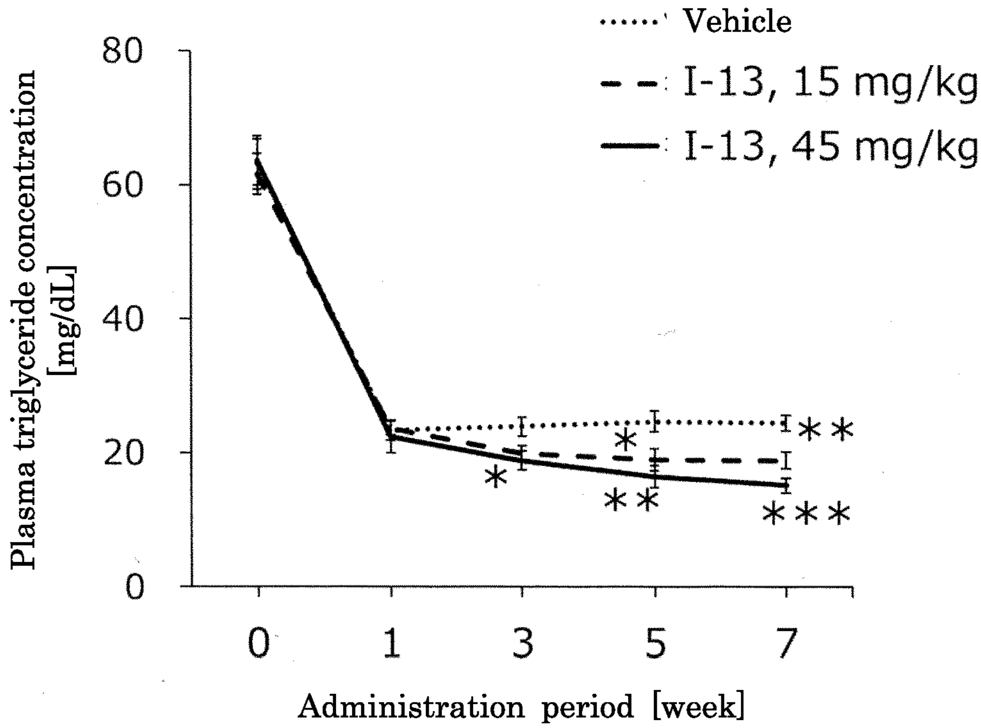


[Fig. 4]

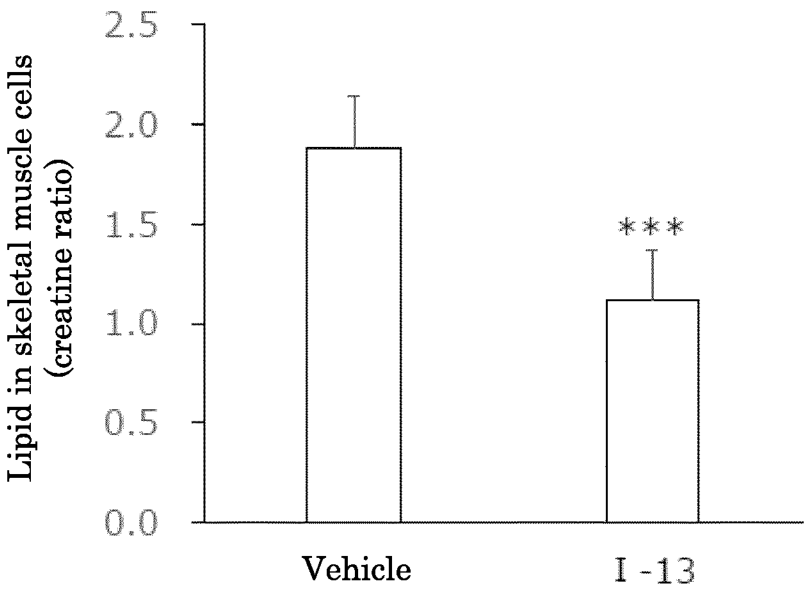


***: $p < 0.001$ vs vehicle-administered group (Welch's t test)

[Fig. 5]

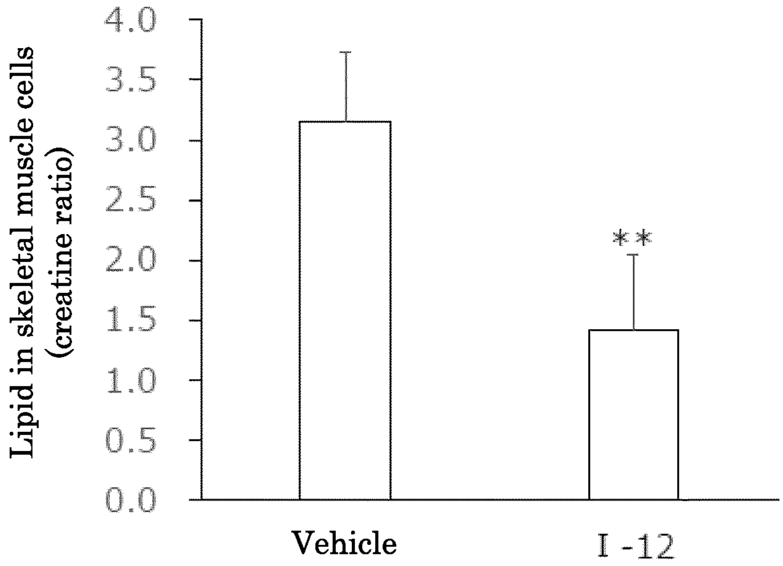


**: $p < 0.01$ vs vehicle-administered group (Welch's t test)

[Fig. 6]

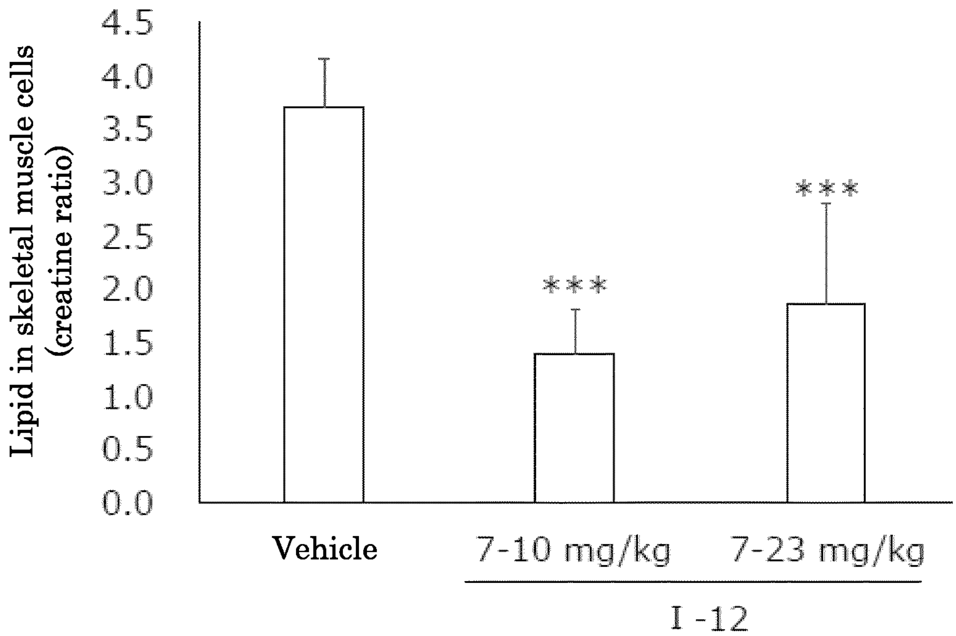


***: $p < 0.001$ vs vehicle-administered group (Dunnett's test)

[Fig. 7]

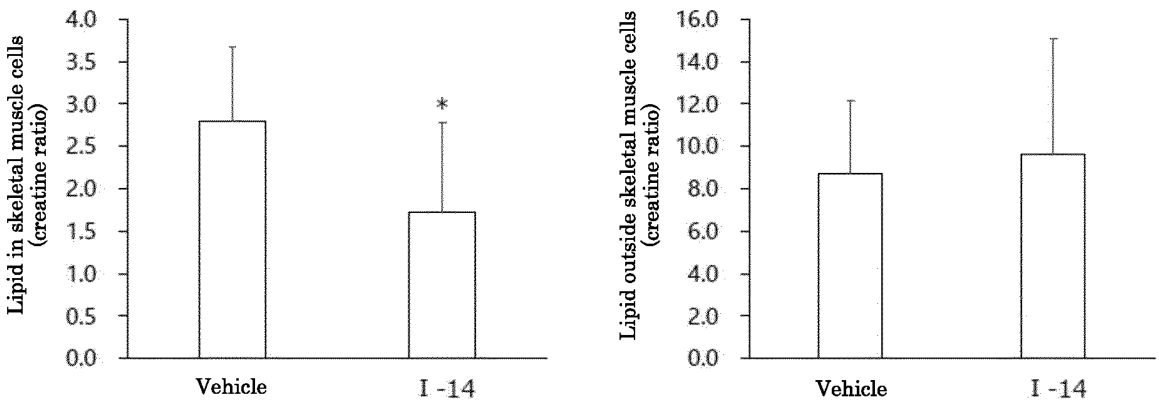


*: $p < 0.05$ vs vehicle-administered group (Welch's t test)

[Fig. 8]

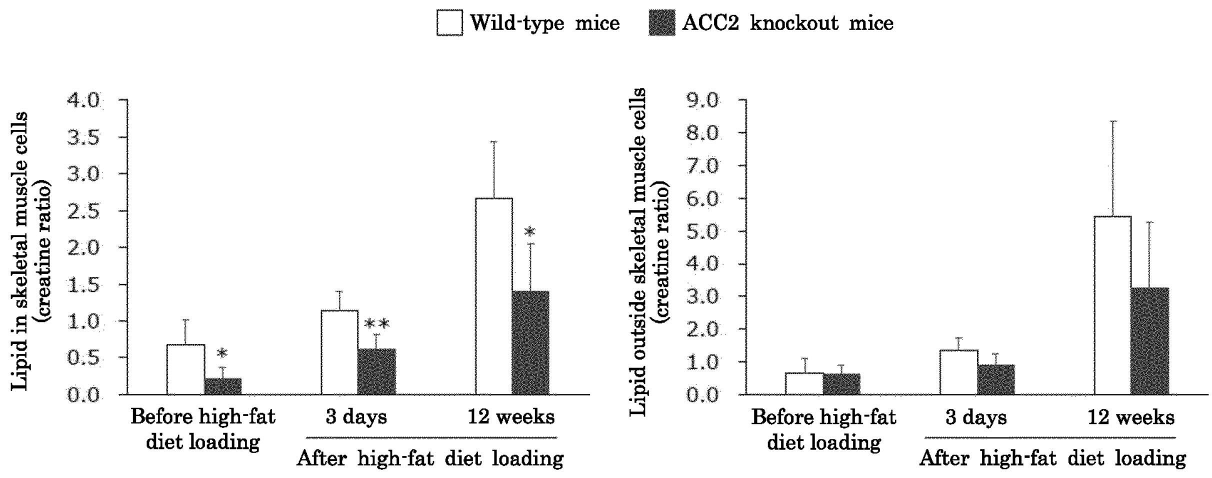


*: $p < 0.05$, **: $p < 0.01$ vs wild-type mice (Welch's t test)

[Fig. 9]

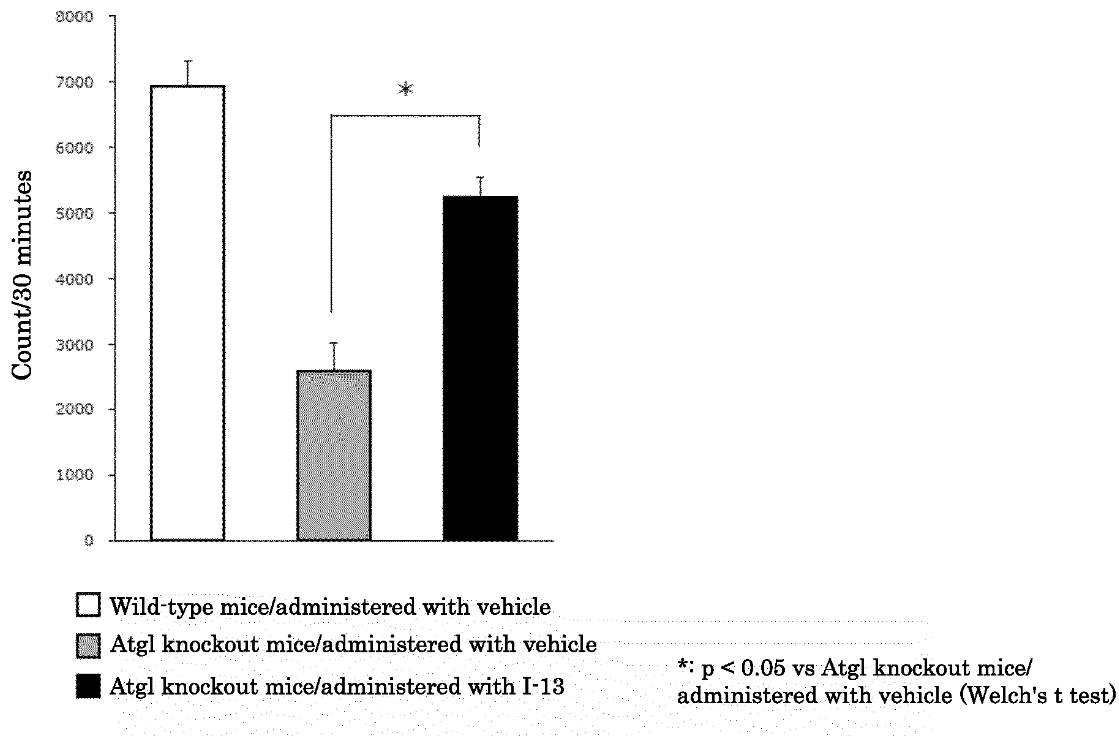
8000
7000
6000
5000
4000
3000
2000
1000
0
Count/30 minutes
*
Wild-type mice/administered with vehicle
Atgl knockout mice/administered with vehicle
Atgl knockout mice/administered with I-13
*: p < 0.05 vs Atgl knockout mice/ administered with vehicle (Welch's t test)

[Fig. 10]

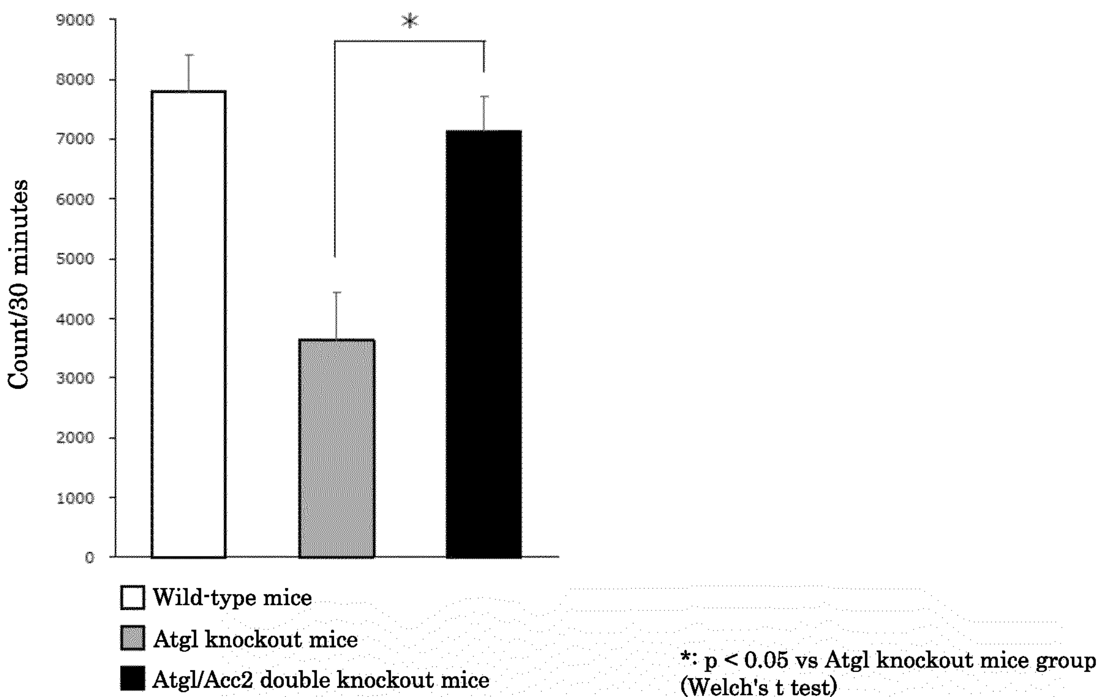
9000
8000
7000
6000
5000
4000
3000
2000
1000
0
Count/30 minutes
*
Wild-type mice
Atgl knockout mice
Atgl/Acc2 double knockout mice
*: p < 0.05 vs Atgl knockout mice group (Welch's t test)

## INTERNATIONAL SEARCH REPORT

International application No.
**PCT/JP2022/042796**

**A. CLASSIFICATION OF SUBJECT MATTER**

***A61K 31/4184***(2006.01)i; ***A61P 3/06***(2006.01)i; ***A61P 3/10***(2006.01)i; ***A61P 7/00***(2006.01)i; ***A61P 9/00***(2006.01)i; ***A61P 9/04***(2006.01)i; ***A61P 9/06***(2006.01)i; ***A61P 9/10***(2006.01)i; ***A61P 13/12***(2006.01)i; ***A61P 21/00***(2006.01)i; ***A61P 43/00***(2006.01)i

FI: A61K31/4184; A61P9/00; A61P9/04; A61P9/10; A61P9/06; A61P21/00; A61P13/12; A61P7/00; A61P43/00 105; A61P3/10; A61P3/06

According to International Patent Classification (IPC) or to both national classification and IPC

**B. FIELDS SEARCHED**

Minimum documentation searched (classification system followed by classification symbols)

A61K31/4184; A61P3/06; A61P3/10; A61P7/00; A61P9/00; A61P9/04; A61P9/06; A61P9/10; A61P13/12; A61P21/00; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2015/056782 A1 (SHIONOGI & CO., LTD.) 23 April 2015 (2015-04-23) entire text | 1-21, 23-29 |
| A | entire text | 22 |
| X | WO 2016/159082 A1 (SHIONOGI & CO., LTD.) 06 October 2016 (2016-10-06) entire text | 1-21, 23-29 |
| A | entire text | 22 |
| P, A | WO 2021/235508 A1 (SHIONOGI & CO., LTD.) 25 November 2021 (2021-11-25) entire text | 1-29 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 January 2023** | **31 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/042796**

| Patent document cited in search report | Publication date (day/month/year) | Patent family member(s) | Publication date (day/month/year) |
|---|---|---|---|
| WO 2015/056782 A1 | 23 April 2015 | JP 2019-1806 A<br>US 2016/0257641 A1<br>entire text<br>EP 3059225 A1<br>EP 3670496 A1 | |
| WO 2016/159082 A1 | 06 October 2016 | JP 2016-79168 A<br>US 2018/0079727 A1<br>entire text<br>US 2019/0144396 A1<br>EP 3279187 A1<br>CN 107428700 A<br>KR 10-2017-0131568 A | |
| WO 2021/235508 A1 | 25 November 2021 | TW 202207919 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013031729 A
- WO 2013071169 A
- WO 2016112305 A
- WO 2011058474 A
- WO 2015056782 A
- WO 2016159082 A
- WO 2021235508 A
- WO 2018007430 A
- WO 2015036892 A
- WO 2014061693 A
- WO 2014056771 A
- WO 2013092616 A
- WO 2008079610 A
- WO 2013035827 A
- WO 2010003624 A
- WO 2007095601 A
- US 20060178400
- WO 2011136385 A
- WO 2012074126 A
- WO 2016041845 A
- WO 2007119833 A
- WO 2008090944 A
- WO 2008102749 A
- JP 2009196966 A
- WO 2010050445 A


### Non-patent literature cited in the description


- *N. Engl. J. Med.,* 2008, vol. 359 (22), 2396-2398
- *J. Atheroscler. Thromb.,* 2009, vol. 16 (5), 702-705
- *PNAS,* 23 August 2005, vol. 102 (34), 12011-12016
- *Molecular Diversity,* 2013, vol. 17 (1), 139-149
- *Journal of Medicinal Chemistry,* 2010, vol. 53 (24), 8679-8687
- *J Pharmacol Exp Ther,* 2020, vol. 372, 256-263
- Isotopes in the Physical and Biomedical Sciences. 1987, vol. 1
- Design of Prodrugs. Elsevier, 1985
- **FOLCH et al.** *J Biol Chem.,* May 1957, vol. 226 (1), 497-509